(19) Europäisches Patentamt
European Patent Office
Office européen des brevets

(11) EP 0 712 905 B1

# (12) EUROPÄISCHE PATENTSCHRIFT

(45) Veröffentlichungstag und Bekanntmachung des Hinweises auf die Patenterteilung:
**29.08.2001 Patentblatt 2001/35**

(51) Int Cl.$^7$: **C09B 62/01**, D06P 1/38

(21) Anmeldenummer: **95810702.1**

(22) Anmeldetag: **08.11.1995**

(54) **Reaktivfarbstoffe, ihre Herstellung und Verwendung**

Reactive dyestuffs, their preparation and use

Colorants réactifs, leur procédé de préparation et leur utilisation

(84) Benannte Vertragsstaaten:
**BE CH DE ES FR GB IT LI PT**

(30) Priorität: **17.11.1994 CH 346894**

(43) Veröffentlichungstag der Anmeldung:
**22.05.1996 Patentblatt 1996/21**

(73) Patentinhaber: **Ciba Specialty Chemicals Holding Inc.**
**4057 Basel (CH)**

(72) Erfinder:
- **Deitz, Rolf**
  **D-79400 Kandern (DE)**
- **Müller, Bernhard, Dr.**
  **D-79588 Efringen-Kirchen (DE)**
- **Tzikas, Athanassios, Dr.**
  **CH-4133 Pratteln (CH)**

(56) Entgegenhaltungen:
**CH-A- 487 987**
**CH-A- 684 482**
**DE-A- 3 513 260**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 712 905 B1

Printed by Jouve, 75001 PARIS (FR)

## Beschreibung

Die vorliegende Erfindung betrifft neue Reaktivfarbstoffe, Verfahren zu ihrer Herstellung und ihre Verwendung.

Die Praxis des Färbens mit Reaktivfarbstoffen hat in neuerer Zeit zu erhöhten Anforderungen an die Qualität der Färbungen und die Wirtschaftlichkeit des Färbeprozesses geführt. Infolge dessen besteht weiterhin ein Bedarf nach neuen Reaktivfarbstoffen, welche verbesserte Eigenschaften, insbesondere in bezug auf die Applikation, aufweisen.

Für das Färben werden heute Reaktivfarbstoffe gefordert, die eine ausreichende Substantivität haben und die zugleich eine gute Auswaschbarkeit der nicht fixierten Anteile aufweisen. Sie sollen ferner eine gute färberische Ausbeute aufweisen und hohe Reaktivität besitzen, wobei insbesondere Färbungen mit hohen Fixiergraden geliefert werden sollen. Von den bekannten Farbstoffen werden diese Anforderungen nicht in allen Eigenschaften erfüllt.

Der vorliegenden Erfindung liegt daher die Aufgabe zugrunde, neue, verbesserte Reaktivfarbstoffe für das Färben und Bedrucken von Fasermaterialien zu finden, welche die oben charakterisierten Qualitäten in hohem Masse besitzen. Die neuen Farbstoffe sollten sich vor allem durch hohe Fixierausbeuten und hohe Faser-Farbstoff-Bindungsstabilitäten auszeichnen, und ausserdem sollten die nicht auf der Faser fixierten Anteile leicht auswaschbar sein. Sie sollten ferner Färbungen mit guten Allgemeinechtheiten, beispielsweise Licht- und Nassechtheiten, ergeben.

Es hat sich gezeigt, dass mit den weiter unten definierten neuen reaktiven Farbstoffen die gestellte Aufgabe weitgehend gelöst wird.

Gegenstand der vorliegenden Erfindung sind Verbindungen der Formel

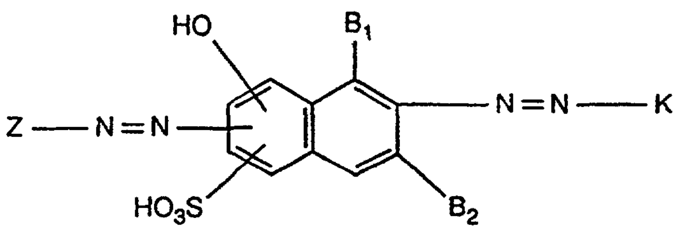

(1),

worin einer der Reste $B_1$ und $B_2$ Wasserstoff und der andere Sulfo ist, K für den Rest einer Kupplungskomponente der Naphthylreihe oder der heterocyclischen Reihe steht und Z den Rest einer hydroxygruppenfreien Diazokomponente der Benzol- oder Naphthalinreihe oder den Rest einer Monoazoverbindung bedeutet, und worin mindestens einer der Reste K oder Z eine faserreaktive Gruppe ausgewählt aus der Gruppe

$$-SO_2-Y \quad (2a),$$

$$-CONR_1-(CH_2)_n-SO_2-Y \quad (2b)$$

und

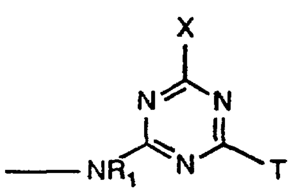

(2c)

aufweist, worin X Halogen, 3-Carboxypyridin-1-yl oder 3-Carbamoylpyridin-1-yl bedeutet, T unabhängig die Bedeutung von X hat oder für Hydroxy, $C_1$-$C_4$-Alkoxy, Phenoxy, $C_1$-$C_4$-Alkylthio, Morpholino, gegebenenfalls durch nicht-reaktive Reste substituiertes Amino oder für einen Reaktivrest der Formel

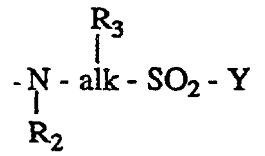 (3a),

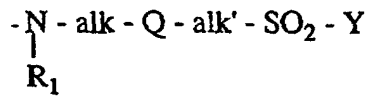 (3b),

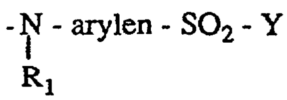 (3c),

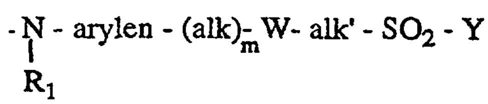 (3d),

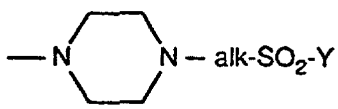 (3e)

oder

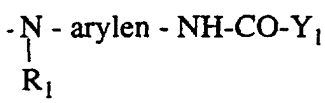 (3f)

steht,

$R_2$ Wasserstoff, gegebenenfalls durch Hydroxy, Sulfo, Sulfato, Carboxy oder Cyano substituiertes $C_1$-$C_4$-Alkyl oder einen Rest

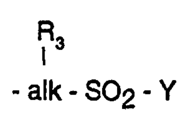

bedeutet,
$R_3$ Wasserstoff, Hydroxy, Sulfo, Sulfato, Carboxy, Cyano, Halogen, $C_1$-$C_4$-Alkoxycarbonyl, $C_1$-$C_4$-Alkanoyloxy, Carbamoyl oder die Gruppe -$SO_2$-Y ist,
alk und alk' unabhängig voneinander $C_1$-$C_6$-Alkylen sind,
arylen einen unsubstituierten oder durch Sulfo, Carboxy, $C_1$-$C_4$-Alkyl, $C_1$-$C_4$-Alkoxy oder Halogen substituierten Phenylen- oder Naphthylenrest bedeutet,
$Y_1$ für eine Gruppe -$CHX_2$-$CH_2X_2$ oder -$CX_2$=$CH_2$ steht und $X_2$ Chlor oder Brom bedeutet,
Q den Rest -O- oder -$NR_1$- bedeutet,
W für eine Gruppe -$SO_2$-$NR_2$-, -$CONR_2$- oder -$NR_2$CO- steht,
m die Zahl 0 oder 1 ist

$R_1$ Wasserstoff oder $C_1$-$C_4$-Alkyl ist,
Y Vinyl oder einen Rest -$CH_2$-$CH_2$-U bedeutet und U eine alkalisch abspaltbare Abgangsgruppe ist, und
n für eine ganze Zahl von 1 bis 6 steht.

Die CH-A-487 987 offenbart Azofarbstoffe, die an der Diazokomponente eine Hydroxygruppe enthalten und in Gegenwart metallabgebender Mittel Metallkomplexazofarbstoffe ergeben.

Die Verbindungen der Formel (1) weisen mindestens zwei Sulfogruppen auf, die jeweils sowohl in Form der freien Säure -$SO_3H$ als auch in einer beliebigen Salzform, z.B. in Form eines Alkali-, Erdalkali- oder Ammoniumsalzes oder in Form eines Salzes eines organischen Amins vorliegen können. Beispiele sind das Natrium-, Kalium-, Lithium- oder Ammoniumsalz, das Salz des Mono-, Di- oder Triethanolamins oder beliebige Na/Li- oder Na/Li/$NH_4$-Mischsalze.

$C_1$-$C_4$-Alkyl bedeutet generell Methyl, Ethyl, n- oder iso-Propyl oder n-, iso-, sec.- oder tert.-Butyl. $C_1$-$C_4$-Alkoxy bedeutet generell Methoxy, Ethoxy, n- oder iso-Propoxy oder n-, iso-, sec.- oder tert.-Butoxy und vorzugsweise Methoxy oder Ethoxy. Halogen steht generell z.B. für Brom oder Chlor. Beispiele für $C_2$-$C_4$-Alkanoyloxy sind Acetyl oder Propionyl, Beispiele für $C_1$-$C_4$-Alkoxy Methoxycarbonyl oder Ethoxycarbonyl. Unter $C_1$-$C_6$-Alkylen ist generell geradkettiges oder in beliebiger Weise verzweigtes $C_1$-$C_6$-Alkylen zu verstehen; Beispiele sind Methylen, 1,1- oder 1,2-Ethylen, 1,2- oder 1,3-Propylen, 1,2-, 1,3-, 1,4- oder 2,3-Butylen, 1,4-, 2,3- oder 2,4-Pentylen, 2-Methyl-1,5-pentylen und 1,6-Hexylen, wobei diese Reste wie angegeben substituiert oder mit Ausnahme von Methylen durch ein Heteroatom -O- oder -$NR_1$- unterbrochen sein können.

Unter faserreaktiven Gruppen sind solche zu verstehen, die mit den Hydroxygruppen der Cellulose, den Amino-, Carboxy-, Hydroxy- und Thiolgruppen bei Wolle und Seide, oder mit den Amino- und eventuell Carboxygruppen von synthetischen Polyamiden unter Bildung kovalenter chemischer Bindungen zu reagieren vermögen. Die faserreaktiven Gruppen der Formeln (2a), (2b) und (2c) sind in der Regel direkt oder über ein Brückenglied an den Farbstoffrest gebunden.

Geeignete alkalisch abspaltbare Abgangsgruppen U sind z.B. Halogen wie etwa Chlor oder Brom, Acyloxy wie etwa Acetoxy oder Benzoyloxy, Phosphato, Sulfato oder Thiosulfato.

Beispiele für geeignete Reste Y sind dementsprechend Vinyl, β-Brom- oder β-Chlorethyl, β-Acetoxyethyl, β-Benzoyloxyethyl, β-Phosphatoethyl, β-Sulfatoethyl und β-Thiosulfatoethyl. Y steht bevorzugt für Vinyl oder β-Sulfatoethyl.

$R_1$ bedeutet vorzugsweise Wasserstoff, Methyl oder Ethyl und besonders bevorzugt Wasserstoff.

n steht bevorzugt für die Zahl 2 oder 3 und besonders bevorzugt für die Zahl 2. m bedeutet bevorzugt die Zahl 0.

Steht T für gegebenenfalls durch nicht-reaktive Reste substituiertes Amino, so kann es sich z.B. um Amino, N-$C_1$-$C_4$-Alkylamino oder N,N-Di-$C_1$-$C_4$-Alkylamino, wobei das Alkyl gegebenenfalls z.B. durch Sulfo, Sulfato, Hydroxy, Carboxy oder Phenyl substituiert ist, Cyclohexylamino, N-$C_1$-$C_4$-Alkyl-N-phenylamino oder Phenylamino oder Naphthylamino, wobei das Phenyl oder Naphthyl gegebenenfalls z.B. durch $C_1$-$C_4$-Alkyl, $C_1$-$C_4$-Alkoxy, $C_2$-$C_4$-Alkanoylamino, Carboxy, Sulfo oder Halogen substituiert ist, handeln.

Beispiele für geeignete nicht-reaktive Aminoreste T sind Amino, Methylamino, Ethylamino, β-Hydroxyethylamino, N,N-Di-β-Hydroxyethylamino, β-Sulfoethylamino, N-Methyl-N-β-Sulfoethylamino, N-Methyl-N-β-Hydroxyethylamino, Cyclohexylamino, Morpholino, o-, m- oder p-Chlorphenylamino, o-, m- oder p-Methylphenylamino, o-, m- oder p-Methoxyphenylamino, o-, m- oder p-Sulfophenylamino, Disulfophenylamino, o-Carboxyphenylamino, 1- oder 2-Naphthylamino, 1-Sulfo-2-naphthylamino, 4,8-Disulfo-2-naphthylamino, N-Ethyl-N-phenylamino, N-Methyl-N-phenylamino, Methoxy, Ethoxy, n- oder iso-Propoxy sowie Hydroxy.

Als nicht-reaktiver Rest hat T vorzugsweise die Bedeutung $C_1$-$C_4$-Alkoxy, Hydroxy, Amino, N-Mono- oder N, N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist.

Besondes bevorzugt steht T als nicht-reaktiver Rest für Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist.

Besonders bevorzugte nicht-reaktive Reste T sind Amino, N-Methylamino, N-Ethylamino, N-β-Sulfoethylamino, N-Methyl-N-β-Sulfoethylamino, N-Methyl-N-β-Hydroxyethylamino, Morpholino, Phenylamino, 2-, 3- oder 4-Sulfophenylamino oder N-$C_1$-$C_4$-Alkyl-N-phenylamino.

X bedeutet bevorzugt Halogen, z.B. Fluor, Chlor oder Brom und insbesondere bevorzugt Chlor oder Fluor. $X_2$ bedeutet vorzugsweise Brom.

Bei alk und alk' handelt es sich unabhängig voneinander z.B. um einen Methylen-, Ethylen-, 1,3-Propylen-, 1,4-Butylen-, 1,5-Pentylen- oder 1,6-Hexylenrest oder deren verzweigte Isomere.

Bevorzugt stehen alk und alk' unabhängig voneinander je für einen $C_1$-$C_4$-Alkylenrest und insbesondere be-

vorzugt für einen Ethylenrest oder Propylenrest.

arylen ist vorzugsweise ein unsubstituierter oder z.B. durch Sulfo, Methyl, Methoxy oder Carboxy substituierter 1,3- oder 1,4-Phenylenrest und besonders bevorzugt ein unsubstituierter 1,3- oder 1,4-Phenylenrest.

$R_2$ ist vorzugsweise Wasserstoff oder $C_1$-$C_4$-Alkyl, besonders bevorzugt Wasserstoff, Methyl oder Ethyl und insbesondere bevorzugt Wasserstoff.

$R_3$ bedeutet bevorzugt Wasserstoff.

Q steht vorzugsweise für -NH- oder -O- und insbesondere bevorzugt für -O-.

W bedeutet bevorzugt eine Gruppe der Formel -CONH- oder -NHCO-, insbesondere eine Gruppe der Formel -CONH-.

Bevorzugt als Reaktivreste der Formeln (3a) bis (3f) sind solche, worin W eine Gruppe der Formel -CONH- oder -NHCO-, $R_1$, $R_2$ und $R_3$ je Wasserstoff, Q der Rest -O- oder -NH-, alk und alk' unabhängig voneinander je Ethylen oder Propylen, arylen unsubstituiertes oder durch Methyl, Methoxy, Carboxy oder Sulfo substituiertes Phenylen, Y Vinyl oder β-Sulfatoethyl, $Y_1$ $-CHBr-CH_2Br$ oder $-CBr{=}CH_2$ und m die Zahl 0 bedeuten.

Weist Z eine faserreaktive Gruppe auf, so entspricht diese bevorzugt der zuvor angegebenen Formel (2a), (2b) oder (2c), worin Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl bedeutet, $R_1$ Wasserstoff, Methyl oder Ethyl ist, n für die Zahl 2 oder 3 steht, X Halogen ist und T $C_1$-$C_4$-Alkoxy, Hydroxy, Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Alkyl gegebenenfalls durch Hydroxy, Sulfo oder Sulfato und das Phenyl jeweils unsubstituiert oder durch Sulfo, Carboxy, Acetylamino, Methyl oder Methoxy substituiert ist, bedeutet.

Ein in Z enthaltener Reaktivrest entspricht besonders bevorzugt der zuvor angegebenen Formel (2a) oder (2c), worin Y Vinyl oder β-Sulfatoethyl bedeutet, $R_1$ Wasserstoff ist, X Chlor oder Fluor ist und T Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, bedeutet.

Weist der Rest K einen Reaktivrest auf, handelt es sich bevorzugt um einen Rest der zuvor angegebenen Formel (2a), (2b) oder (2c), worin Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl bedeutet, $R_1$ Wasserstoff, Methyl oder Ethyl ist, n für die Zahl 2 oder 3 steht, X Halogen ist und T $C_1$-$C_4$-Alkoxy, Hydroxy, Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Alkyl gegebenenfalls durch Hydroxy, Sulfo oder Sulfato und das Phenyl jeweils unsubstituiert oder durch Sulfo, Carboxy, Acetylamino, Methyl oder Methoxy substituiert ist, oder einen faserreaktiven Rest der Formel

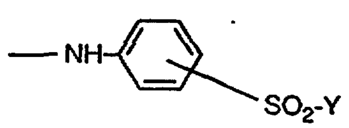 (3c')

oder

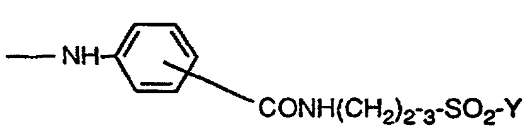 (3d'),

worin Y die zuvor angegebene Bedeutung hat, bedeutet.

Die Reste K und Z können einen oder auch mehrere gleiche oder verschiedene faserreaktive Gruppen aufweisen.

K bedeutet z.B. den Rest einer Kupplungskomponente der Naphthalin-, Phenylazonaphthalin-, 4-Alkyl-6-hydroxypyridon-(2)-, 2,5-Diamino-4-alkylpyridin-, 1-Arylpyrazolon-(5)- oder 1-Aryl-5-aminopyrazol-Reihe, wobei diese farbstoffübliche Substituenten und gegebenenfalls eine oder mehrere faserreaktive Gruppen enthalten kann.

Aus der Reihe der Substituenten seien beispielhaft genannt: Alkylgruppen mit 1 bis 12 Kohlenstoffatomen, insbesondere 1 bis 4 Kohlenstoffatomen, wie Methyl, Ethyl, n- oder iso-Propyl, oder n-, iso-, sec.- oder tert.-Butyl, Alkoxygruppen mit 1 bis 8 Kohlenstoffatomen, insbesondere 1 bis 4 Kohlenstoffatomen, wie Methoxy, Ethoxy, n- oder iso-Propoxy, oder n-, iso-, sec.- oder tert.-Butoxy, im Alkylteil z.B. durch Hydroxy, $C_1$-$C_4$-Alkoxy oder Sulfato substituiertes $C_1$-$C_4$-Alkoxy wie z.B. 2-Hydroxyethoxy, 3-Hydroxypropoxy, 2-Sulfatoethoxy, 2-Methoxyethoxy oder 2-Ethoxyethoxy, Acylaminogruppen mit 2 bis 8 Kohlenstoffatomen, insbesondere $C_2$-$C_4$-Alkanoylaminogruppen wie Acetylamino oder Propionylamino, Benzoylamino oder $C_2$-$C_4$-Alkoxycarbonylaminogruppen wie Methoxycarbonylamino oder Ethoxycarbonylamino, Amino, gegebenenfalls im Alkylteil z.B. durch Hydroxy, Sulfo, Sulfato oder $C_1$-$C_4$-Alkoxy substituiertes N-Mono- oder N,N-Di-$C_1$-$C_4$-Alkylamino, z.B. Methylamino, Ethylamino, N,N-Dimethyl- oder N,N-Diethylamino, Sulfomethylamino, β-Hydroxyethylamino, N,N-Di-(2-hydroxyethylamino), N-β-Sulfatoethylamino, gegebenenfalls im Phenylteil durch Methyl, Methoxy, Halogen oder Sulfo substituiertes Phenylamino, gegebenenfalls im Alkylteil durch Hydroxy, Sulfo oder Sulfato oder im Phenylteil gegebenenfalls durch Methyl, Methoxy, Halogen oder Sulfo substituiertes N-$C_1$-$C_4$-Alkyl-N-phenylamino, z.B. N-Methyl-N-phenylamino, N-Ethyl-N-phenylamino, N-β-Hydroxyethyl-N-phenylamino oder N-β-Sulfoethyl-N-phenylamino, gegebenenfalls durch Sulfo substituiertes Naphthylamino, Alkanoylgruppen mit 2 bis 8 Kohlenstoffatomen, insbesondere 2 bis 4 Kohlenstoffatomen, z.B. Acetyl oder Propionyl, Benzoyl, Alkoxycarbonyl mit 1 bis 4 Kohlenstoffatomen im Alkoxyrest, wie Methoxycarbonyl oder Ethoxycarbonyl, Alkylsulfonyl mit 1 bis 4 Kohlenstoffatomen, wie Methylsulfonyl oder Ethylsulfonyl, Phenyl- oder Naphthylsulfonyl, Trifluormethyl, Nitro, Cyano, Hydroxyl, Halogen, wie Fluor, Chlor oder Brom, Carbamoyl, N-$C_1$-$C_4$-Alkylcarbamoyl, wie N-Methylcarbamoyl oder N-Ethylcarbamoyl, Sulfamoyl, N-$C_1$-$C_4$-Alkylsulfamoyl wie N-Methylsulfamoyl, N-Ethylsulfamoyl, N-Propylsulfamoyl, N-Isopropylsulfamoyl oder N-Butylsulfamoyl, N-(β-Hydroxyethyl)-sulfamoyl, N,N-Di-(β-hydroxyethyl)-sulfamoyl, N-Phenylsulfamoyl, Ureido, Carboxy, Sulfomethyl, Sulfo, Sulfato, ein Rest der Formel

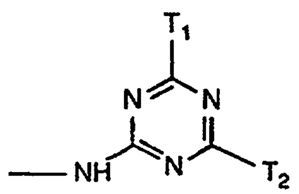

(2c'),

worin $T_1$ und $T_2$ unabhängig voneinander je für Hydroxy, $C_1$-$C_4$-Alkoxy, Morpholino oder gegebenenfalls durch nichtreaktive Reste substituiertes Amino stehen, sowie die zuvor genannten faserreaktiven Reste. Die Alkylreste können zudem durch Sauerstoff (-O-) oder -$NR_1$-, worin $R_1$ die zuvor angegebene Bedeutung hat, unterbrochen sein. In Formel (2c') stehen $T_1$ und $T_2$ unabhängig voneinander je bevorzugt für Amino, N-Mono- oder N,N-Di-$C_1$-$C_4$-Alkylamino, worin das Alkyl unsubstituiert oder durch Hydroxy, Sulfato oder Sulfo substituiert ist, Morpholino oder Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist.

K steht z.B. für einen Rest der Formel

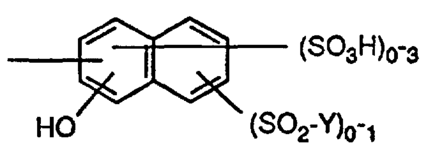

(4a)

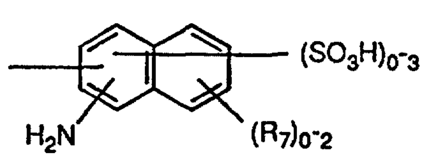

(4b)

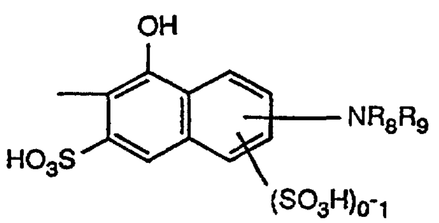

(4c)

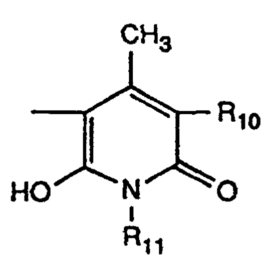

(4d)

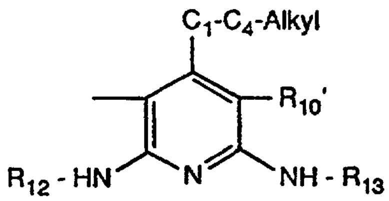 (4e) 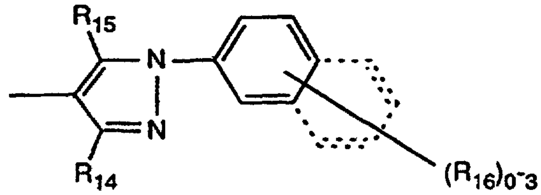 (4f)

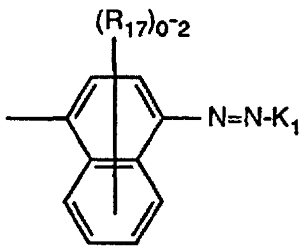 (4g) 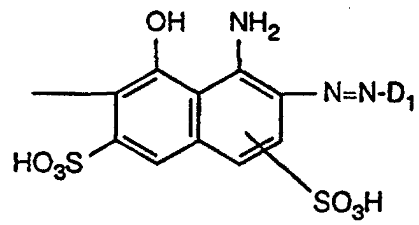 (4h)

oder

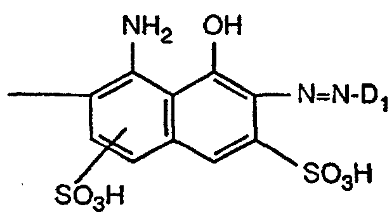 (4i),

worin $(R_7)_{0-2}$ für 0 bis 2 gleiche oder verschiedene Substituenten $R_7$ aus der Gruppe Hydroxy und faserreaktiver Rest der zuvor angegebenen Formel (2a) steht;

$R_8$ Wasserstoff oder gegebenenfalls durch Hydroxy, $C_1$-$C_4$-Alkoxy, Sulfato, Sulfo, Halogen oder Cyano substituiertes $C_1$-$C_4$-Alkyl ist;
$R_9$ unabhängig die Bedeutung von $R_8$ hat oder $C_2$-$C_4$-Alkanoyl, Benzoyl oder einen faserreaktiven Rest der zuvor angegebenen Formel (2c) bedeutet;
$R_{10}$ und $R_{10}'$ unabhängig voneinander je Carbamoyl, Sulfomethyl oder Cyano sind;
$R_{11}$ für Wasserstoff oder $C_1$-$C_4$-Alkyl, welches gegebenenfalls durch einen Rest der zuvor angegebenen Formel (2c) substituiert ist, steht;
$R_{12}$ und $R_{13}$ unabhängig voneinander je für Wasserstoff oder gegebenenfalls durch Hydroxy, $C_1$-$C_4$-Alkoxy, Phenoxy, Sulfato, Sulfo, Carboxy, Amino oder N-Mono- oder N,N-Di-$C_1$-$C_4$-Alkylamino, welches seinerseits im Alkylteil durch Hydroxy,
$C_1$-$C_4$-Alkoxy, Phenoxy, Sulfato, Sulfo, Carboxy oder einen faserreaktiven Rest der zuvor angegebenen Formel (2c) substituiert sein kann, substituiertes und/oder mit Ausnahme von Methyl gegebenenfalls durch -O- unterbrochenes $C_1$-$C_{12}$-Alkyl stehen;
$R_{14}$ Methyl oder Carboxy bedeutet;
$R_{15}$ Hydroxy oder Amino ist;
$(R_{16})_{0-3}$ für 0 bis 3 gleiche oder verschiedene Reste $R_{16}$ aus der Gruppe Sulfo, Halogen, Hydroxy, $C_1$-$C_4$-Alkoxy und $C_1$-$C_4$-Alkyl steht;
$(R_{17})_{0-2}$ für 0 bis 2 gleiche oder verschiedene Reste $R_{17}$ aus der Gruppe $C_1$-$C_4$-Alkyl, $C_1$-$C_4$-Alkoxy, im Alkylteil durch Hydroxy, $C_1$-$C_4$-Alkoxy oder Sulfato substituiertes $C_1$-$C_4$-Alkoxy, $C_2$-$C_4$-Alkanoylamino, Ureido, Halogen und Sulfo steht;
$K_1$ unabhängig einen Rest der zuvor angegebenen Formel (4a) - (4f), (4h) oder (4i) und vorzugsweise einen Rest der Formel (4c) bedeutet;
$D_1$ ein Phenyl- oder 1- oder 2-Naphthylrest ist, der 1 bis 3 gleiche oder verschiedene Reste aus der Gruppe Sulfo,

$C_1$-$C_4$-Alkyl, $C_1$-$C_4$-Alkoxy, Halogen oder faserreaktiver Rest der Formel (2a), (2b) oder (2c) trägt; und X und Y jeweils die zuvor angegebene Bedeutung haben.

Für die in den Formeln (4a) bis (4i) angegebenen Variablen gelten die folgenden Bevorzugungen:

$R_8$ ist vorzugsweise Wasserstoff;
$R_9$ bedeutet vorzugsweise Acetyl, Propionyl, Benzoyl oder einen faserreaktiven Rest der zuvor angegebenen Formel (2c);
$R_{10}$ steht bevorzugt für Carbamoyl oder Sulfomethyl;
$R_{10}'$ steht bevorzugt für Cyano oder Carbamoyl;
$R_{11}$ bedeutet bevorzugt Methyl oder Ethyl;
$R_{12}$ steht bevorzugt für Wasserstoff oder gegebenenfalls durch Hydroxy, Methoxy, Ethoxy, Sulfato oder Sulfo, substituiertes $C_1$-$C_6$-Alkyl;
$R_{13}$ hat vorzugsweise unabhängig die Bedeutung von $R_{12}$ oder steht für einen Rest der zuvor angegebenen Formel (2c);
$R_{15}$ bedeutet bevorzugt Hydroxy;
$R_{12}$ und $R_{13}$ stehen unabhängig voneinander je besonders bevorzugt für Wasserstoff oder unsubstituiertes oder durch Hydroxy, Sulfo oder Sulfato substituiertes $C_1$-$C_6$-Alkyl;
$(R_{16})_{0-3}$ steht bevorzugt für 0 bis 3 gleiche oder verschiedene Reste $R_{16}$ aus der Gruppe Sulfo, Methyl, Methoxy, Hydroxy und Chlor;
$(R_{17})_{0-2}$ bedeutet bevorzugt 0 bis 2 gleiche oder verschiedene Reste $R_{17}$ aus der Gruppe Methyl, Methoxy, im Alkylteil durch Hydroxy oder Sulfato substituiertes $C_1$-$C_2$-Alkoxy, Acetylamino, Propionylamino und Sulfo; und
$D_1$ ist bevorzugt ein Phenyl- oder 1- oder 2-Naphthylrest, der 1 bis 3 gleiche oder verschiedene Reste aus der Gruppe Sulfo, Methyl, Methoxy, Chlor und faserreaktiver Rest der Formel (2a) oder (2c) trägt;

K steht bevorzugt für einen Rest der Formel

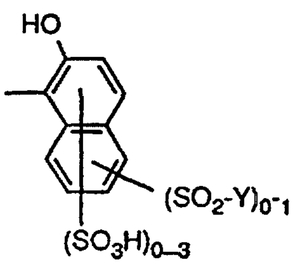 (4a'),

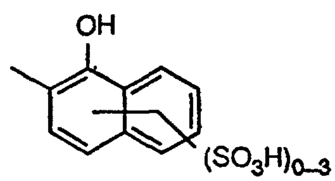 (4a''),

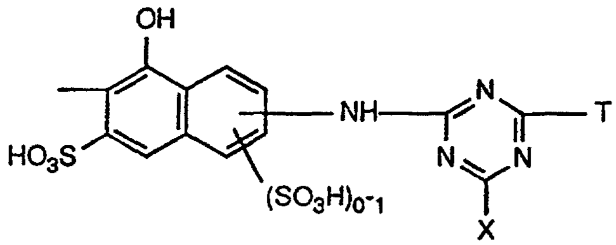 , (4c')

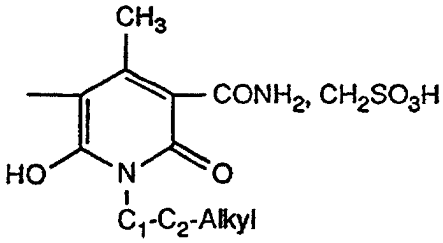 (4d'), 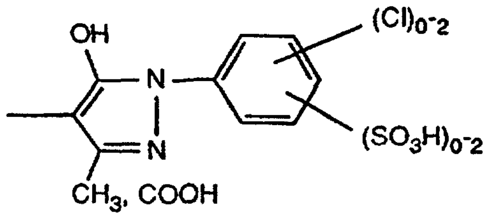 (4f'),

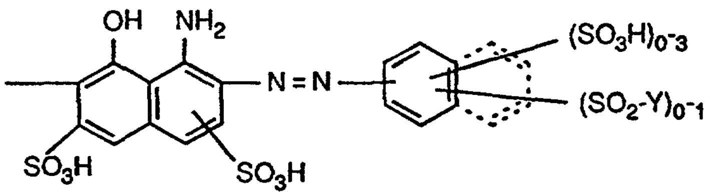 (4h')

oder

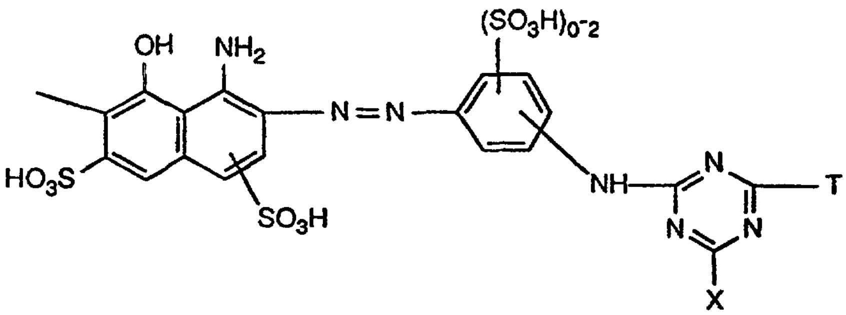 (4h'')

worin X Chlor oder Fluor ist, T Amino, N-Mono- oder N,N-Di-$C_1$-$C_4$-Alkylamino, worin das Alkyl unsubstituiert oder durch Hydroxy, Sulfato oder Sulfo substituiert ist, Morpholino oder Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, bedeutet und für Y die zuvor angegebenen Bedeutungen und Bevorzugungen gelten.

K steht besonders bevorzugt für einen Rest der zuvor angegebenen Formel (4c'), (4h') oder (4h").

Z bedeutet vorzugsweise einen Phenyl-, Naphthyl-, Phenylazo- oder Naphthylazophenylrest, der eine oder mehrere der zuvor genannten faserreaktiven Gruppen der Formel (2a), (2b) oder (2c) sowie die bei organischen Farbstoffen üblichen Substituenten, wie sie z.B. zuvor für K genannt wurden, aufweisen kann.

Beispiele für bevorzugte Substituenten am Rest Z sind $C_1$-$C_4$-Alkyl, $C_1$-$C_4$-Alkoxy, Halogen, Sulfo oder ein Rest der zuvor angegebenen Formel (2c') sowie die zuvor genannten faserreaktiven Gruppen.

Z steht besonders bevorzugt für einen Phenyl-, Naphthyl-, oder Phenylazophenylrest, der eine faserreaktive Gruppe der Formel (2a) und/oder (2c) trägt, worin Y Vinyl oder β-Sulfatoethyl, $R_1$ Wasserstoff, X Chlor oder Fluor und T Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, bedeuten, und weiter unsubstituiert oder durch Methyl, Ethyl, Methoxy, Ethoxy, Chlor, Sulfo oder einen Rest der zuvor angegebenen Formel (2c'), worin für $T_1$ und $T_2$ die zuvor genannten Bedeutungen und Bevorzugungen gelten, substituiert ist.

Z steht insbesondere bevorzugt für einen Rest der Formel

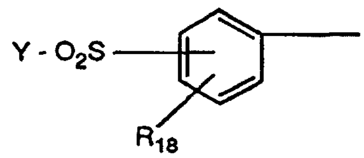

(5a),

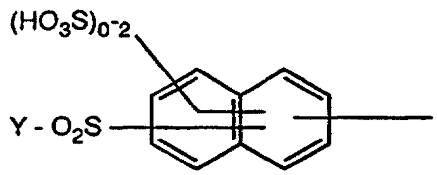

(5b)

oder

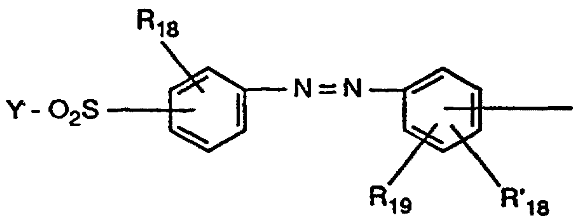

(5c),

worin $R_{18}$ und $R'_{18}$ unabhängig voneinander je Wasserstoff, Sulfo, Methyl, Ethyl, Methoxy, Ethoxy oder Chlor bedeuten, $R_{19}$ Wasserstoff, Sulfo oder ein Rest der zuvor angegebenen Formel (2c) oder (2c'), worin für $R_1$, X, T, $T_1$ und $T_2$ die zuvor genannten Bedeutungen und Bevorzugungen gelten, ist und für Y die zuvor genannten Bedeutungen und Bevorzugungen gelten.

Eine bevorzugte Ausführungsform der vorliegenden Erfindung stellen Verbindungen der Formel

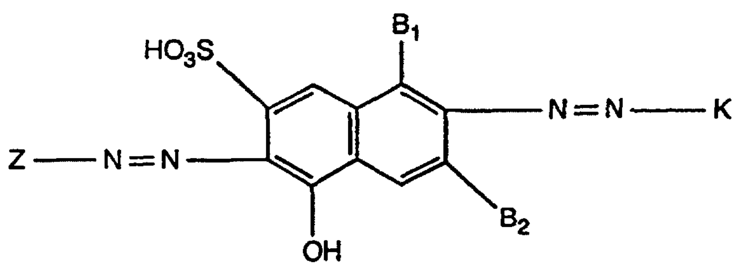

(1a)

dar, worin einer der Reste $B_1$ und $B_2$ Wasserstoff und der andere Sulfo bedeuten und für die Variablen K und Z die zuvor genannten Bedeutungen und Bevorzugungen gelten. Besonders bevorzugt sind dabei Verbindungen der Formel (1a), worin $B_1$ Sulfo und $B_2$ Wasserstoff bedeuten.

Eine weitere bevorzugte Ausführungsform der vorliegenden Erfindung stellen Verbindungen der Formel

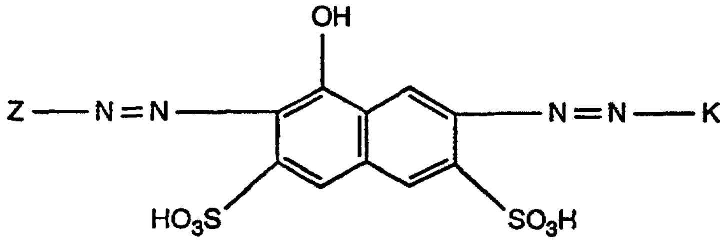

(1b)

dar, worin für die Variablen K und Z die zuvor genannten Bedeutungen und Bevorzugungen gelten.

Die Verbindungen der Formel (1) bzw. (1a) oder (1b) können erhalten werden, z.B. indem man in etwa 1 Moläquivalent einer Verbindung der Formel

$$Z\text{-}NH_2 \quad (6)$$

in üblicher Weise, z.B. mit Nitriten wie Natriumnitrit im sauren, z.B. salzsauren, Medium diazotiert und mit etwa einem Moläquivalent einer Verbindung der Formel

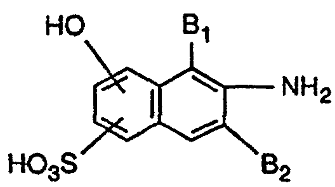

(7)

kuppelt, und die erhaltene Monoazoverbindung erneut in üblicher Weise diazotiert und mit einer Verbindung der Formel

$$K\text{ - }H \quad (8)$$

kuppelt, wobei Z, $B_1$, $B_2$ und K jeweils die zuvor angegebene Bedeutung haben.

Die beiden obengenannten Kupplungsreaktionen werden z.B. bei einem neutralen bis leicht sauren pH-Wert, z.B. bei pH 3 bis 7, und Temperaturen von -5 bis 30°C durchgeführt.

Die Verbindungen der Formeln (6) bis (8) sind bekannt oder können in an sich bekannter Weise erhalten werden.

Die Verbindungen der Formel (1) eignen sich als Reaktivfarbstoffe zum Färben und Bedrucken der verschiedensten Materialien, wie Seide, Leder, Wolle, Polyamidfasern und Polyurethanen, und insbesondere von cellulosehaltigen Fasermaterialien aller Art. Solche Fasermaterialien sind beispielsweise die natürliche Cellulosefaser, wie Baumwolle, Leinen und Hanf, sowie Zellstoff und regenerierte Cellulose. Die Reaktivfarbstoffe der Formel (1) sind auch zum Färben oder Bedrucken von hydroxylgruppenhaltigen Fasern geeignet, die in Mischgeweben enthalten sind, z.B. von Gemischen aus Baumwolle mit Polyesterfasern oder Polyamidfasern.

Die erfindungsgemässen Farbstoffe lassen sich auf verschiedene Weise auf das Fasermaterial applizieren und auf der Faser fixieren, insbesondere in Form von wässrigen Farbstofflösungen und -druckpasten. Sie eignen sich sowohl für das Ausziehverfahren als auch zum Färben nach dem Foulardverfahren, können bei niedrigen Färbetemperaturen eingesetzt werden und erfordern bei Pad-Steam-Verfahren nur kurze Dämpfzeiten. Die Fixiergrade sind hoch, und die nicht fixierten Anteile können leicht ausgewaschen werden, wobei die Differenz zwischen Ausziehgrad und Fixiergrad bemerkenswert klein, d.h. der Seifverlust sehr gering ist. Die Reaktivfarbstoffe der Formel (1) eignen sich auch zum Druck, vor allem auf Baumwolle, ebenso aber auch zum Bedrucken von stickstoffhaltigen Fasern, z.B. von Wolle, Seide oder Wolle enthaltenden Mischgeweben.

Die mit den erfindungsgemässen Farbstoffen hergestellten Färbungen und Drucke auf Cellulosefasermaterialien besitzen eine hohe Farbstärke und eine hohe Faser-Farbstoff-Bindungsstabilität, sowohl in saurem als auch in alkalischem Bereich, weiterhin eine gute Lichtechtheit und sehr gute Nassechtheitseigenschaften, wie Wasch-, Wasser-, Seewasser-, Überfärbe- und Schweissechtheiten, sowie eine gute Plissierechtheit, Bügelechtheit, Reibechtheit und insbesondere Chlorechtheit.

Die nachfolgenden Beispiele dienen zur Erläuterung der Erfindung. Die Temperaturen sind in Celsiusgraden angegeben, Teile sind Gewichtsteile, die Prozentangaben beziehen sich auf Gewichtsprozente, sofern nicht anderes vermerkt. Gewichtsteile stehen zu Volumenteilen im Verhältnis von Kilogramm zum Liter.

Beispiel 1: 28 Teile 2-(4-Aminophenylsulfonyl)-ethyl-hydrogensulfat werden in 150 Teile Wasser eingetragen und gut verrührt. Zu dieser Suspension gibt man 16 Teile konz. Salzsäure, kühlt auf 0 bis 5°C ab und tropft langsam 7 Teile Natriumnitrit, gelöst in 25 Teilen Wasser, zu. Nach erfolgter Diazotierung wird das überschüssige Nitrit mit Sulfaminsäure zerstört.

Die solchermassen bereitete Diazolösung wird anschliessend bei 0 bis 5°C zu einer Lösung von 32 Teilen 6-Amino-1-hydroxynaphthalin-3,5-disulfonsäure in 150 Teilen Wasser gegeben und dabei der pH-Wert durch Zugabe von Natriumhydroxidlösung konstant bei pH 7 gehalten.

Nachdem die Kupplung vollständig ist, wird die erhaltene Aminoazoverbindung der Formel

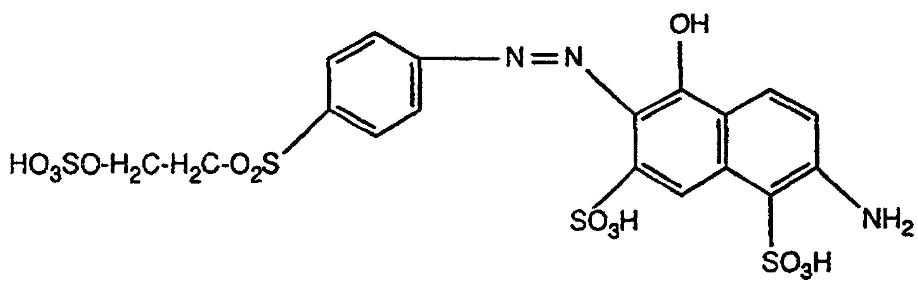

ohne vorherige Isolierung erneut bei 0 bis 5°C mit 7 Teilen Natriumnitrit in salzsaurer Lösung diazotiert und der Nitritüberschuss am Ende mit Sulfaminsäure zerstört (Diazolösung 1).

Beispiele 2-3: Verfährt man wie im Beispiel 1 beschrieben und verwendet anstelle von 6-Amino-1-hydroxynaphthalin-3,5-disulfonsäure eine äquivalente Menge 6-Amino-1-hydroxynaphthalin-3,7-disulfonsäure oder 7-Amino-1-hydroxynaphthalin-3,6-disulfonsäure, erhält man Lösungen der Diazoverbindungen von folgenden Amino-Verbindungen.

| Beispiel Nr. | Aminoverbindung | |
|---|---|---|
| 2 | 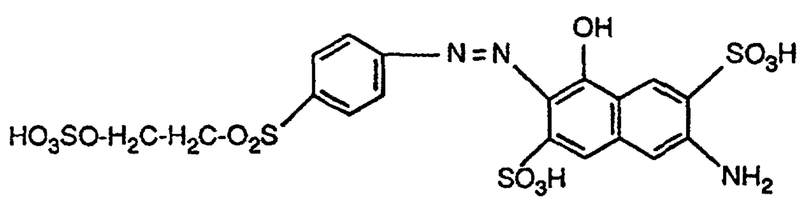 | (Diazolösung 2) |
| 3 | 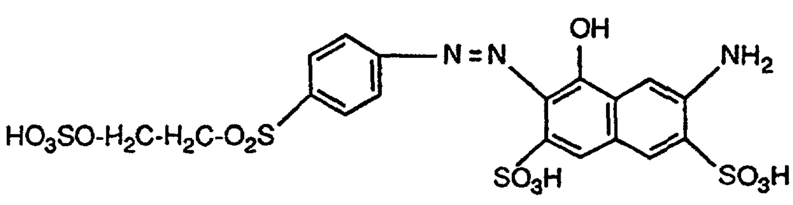 | (Diazolösung 3) |

Beispiel 4: 35 Teile 2-(2-Amino-1-sulfonaphth-6-yl-sulfonyl)-ethyl-hydrogensulfat werden in 200 Teile Wasser eingetragen und gut verrührt. Zu dieser Suspension gibt man 16 Teile konz. Salzsäure, kühlt auf 0 bis 5°C ab und tropft langsam 7 Teile Natriumnitrit, gelöst in 25 Teilen Wasser, zu. Nach erfolgter Diazotierung wird das überschüssige Nitrit mit Sulfaminsäure zerstört.

Die solchermassen bereitete Diazolösung wird anschliessend bei 0 bis 5°C zu einer Lösung von 32 Teilen 6-Amino-1-hydroxynaphthalin-3,5-disulfonsäure in 150 Teilen Wasser gegeben und dabei der pH-Wert durch Zugabe von Natriumhydroxidlösung konstant bei pH 7 gehalten.

Nachdem die Kupplung vollständig ist, wird die erhaltene Aminoazoverbindung der Formel

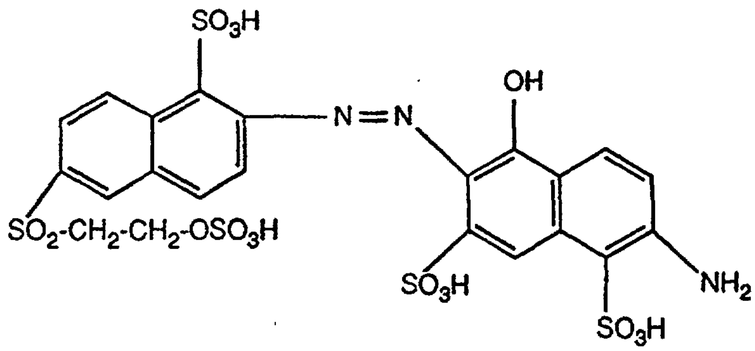

ohne vorherige Isolierung erneut bei 0 bis 5°C mit 7 Teilen Natriumnitrit in salzsaurer Lösung diazotiert und der Nitritüberschuss am Ende mit Sulfaminsäure zerstört (Diazolösung 4).

Beispiele 5-6: Verfährt man wie im Beispiel 4 beschrieben und verwendet anstelle von 6-Amino-1-hydroxynaphthalin-3,5-disulfonsäure eine äquivalente Menge 6-Amino-1-hydroxynaphthalin-3,7-disulfonsäure oder 7-Amino-1-hydroxynaphthalin-3,6-disulfonsäure, erhält man Lösungen der Diazoverbindungen von folgenden Amino-Verbindungen.

| Beispiel Nr. | Aminoverbindung | |
|---|---|---|
| 5 | 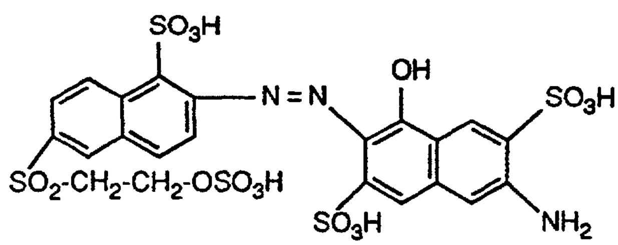 | (Diazolösung 5) |
| 6 | 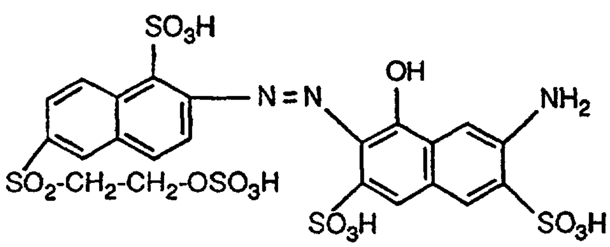 | (Diazolösung 6) |

Beispiel 7: 63 Teile der Verbindung der Formel

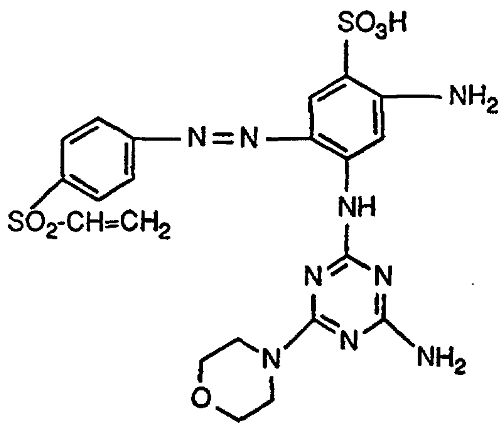

(Herstellung gemäss EP-A 0 647 683) werden in 600 Teile Wasser eingetragen und gut verrührt. Zu dieser Suspension gibt man 16 Teile konz. Salzsäure, kühlt auf 0 bis 5°C ab und tropft langsam 7 Teile Natriumnitrit, gelöst in 25 Teilen Wasser, zu. Nach erfolgter Diazotierung wird das überschüssige Nitrit mit Sulfaminsäure zerstört.

Die solchermassen bereitete Diazolösung wird anschliessend bei 0 bis 5°C zu einer Lösung von 32 Teilen 6-Amino-1-hydroxynaphthalin-3,5-disulfonsäure in 150 Teilen Wasser gegeben und dabei der pH-Wert durch Zugabe von Natriumhydroxidlösung konstant bei pH 7 gehalten.

Nachdem die Kupplung vollständig ist, wird die erhaltene Aminoazoverbindung der Formel

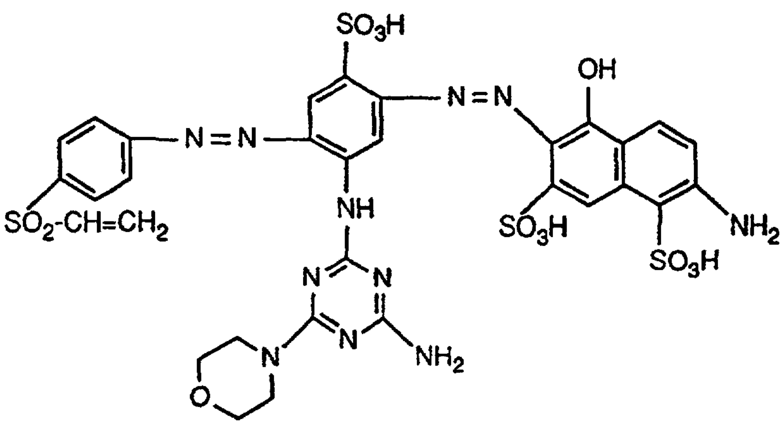

ohne vorherige Isolierung erneut bei 0 bis 5°C mit 7 Teilen Natriumnitrit in salzsaurer Lösung diazotiert und der Nitritüberschuss am Ende mit Sulfaminsäure zerstört (Diazolösung 7).

Beispiele 8-9: Verfährt man wie im Beispiel 7 beschrieben und verwendet anstelle von 6-Amino-1-hydroxynaphthalin-3,5-disulfonsäure eine äquivalente Menge 6-Amino-1-hydroxynaphthalin-3,7-disulfonsäure oder 7-Amino-1-hydroxynaphthalin-3,6-disulfonsäure, erhält man Lösungen der Diazoverbindungen von folgenden Amino-Verbindungen.

| Beispiel Nr. | Aminoverbindung | |
|---|---|---|
| 8 | 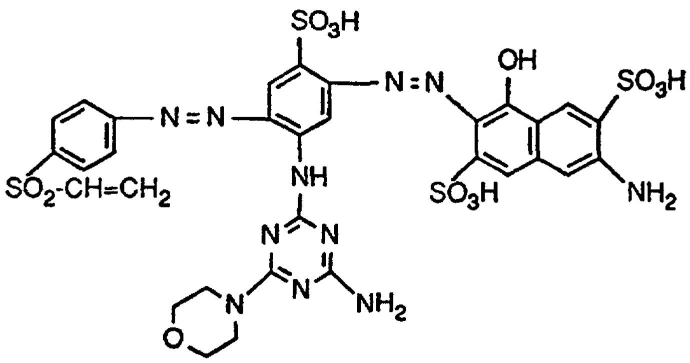 | (Diazolösung 8) |
| 9 | 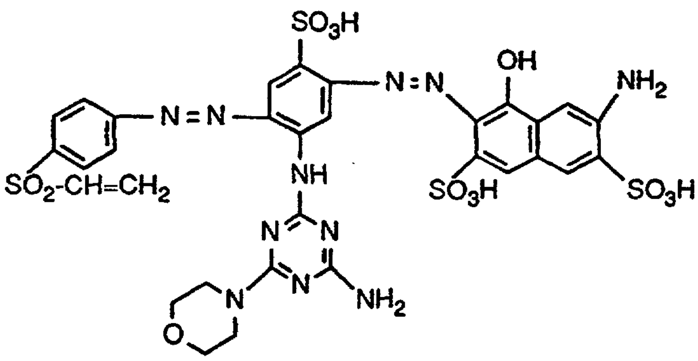 | (Diazolösung 9) |

Beispiele 10-12: Analog wie in den Beispielen 7 bis 9 beschrieben lassen sich die Diazoverbindungen der folgenden Amino-Verbindungen herstellen.

| | | |
|---|---|---|
| 10 | 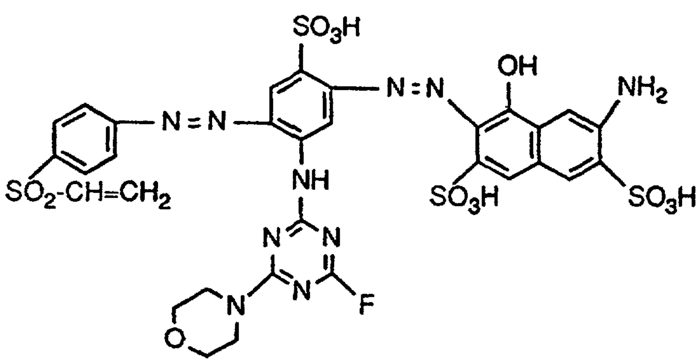 | (Diazolösung 10) |

11 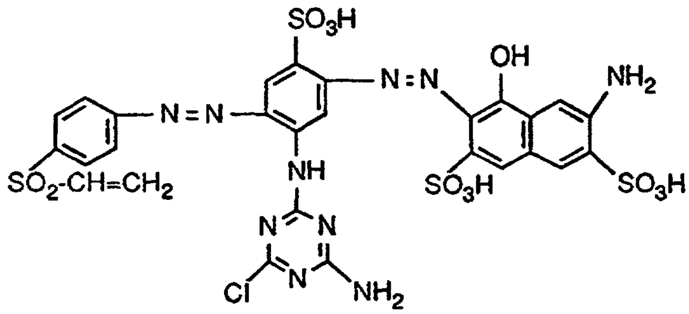 (Diazolösung 11)

12 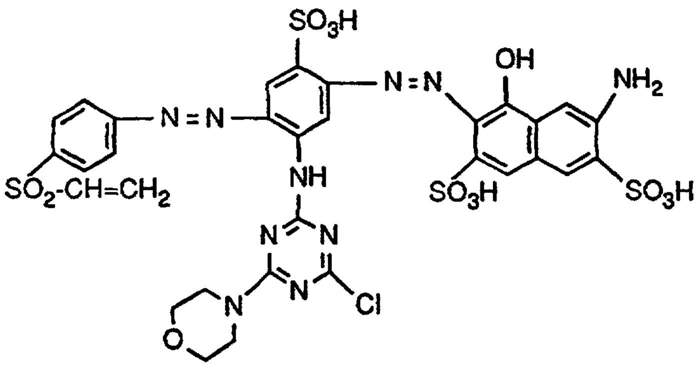 (Diazolösung 12)

Beispiel 13: In die Diazolösung 1 gemäss Beispiel 1 werden bei ca. 5°C 20 Teile 1-Ethyl-4-methyl-5-carbamoyl-6-hydroxypyrid-2-on eingetragen. Danach erhöht man den pH-Wert des Reaktionsgemisches durch Zugabe von Natriumhydroxidlösung allmählich auf 7 und rührt, bis die Kupplung komplett ist. Die Farbstofflösung wird dialytisch von Salz befreit und im Vakuum eingedampft. Man erhält die Verbindung, die in Form der freien Säure der Formel

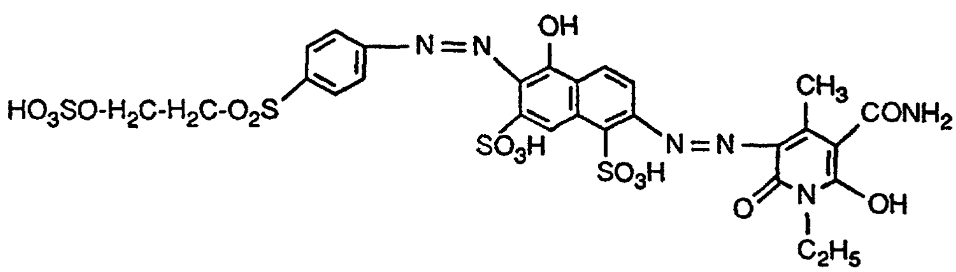

entspricht, als rötliches Pulver, welches Cellulose in orangen Tönen mit guten Allgemeinechtheiten färbt.

Beispiel 14: In die Diazolösung 2 gemäss Beispiel 2 werden bei ca. 5°C 25 Teile 1-(3-sulfophenyl)-3-methylpyrazol-5-on eingetragen. Danach erhöht man den pH-Wert des Reaktionsgemisches durch Zugabe von Natriumhydroxidlösung allmählich auf 7 und rührt, bis die Kupplung komplett ist. Die Farbstofflösung wird dialytisch von Salz befreit und im Vakuum eingedampft. Man erhält die Verbindung, die in Form der freien Säure der Formel

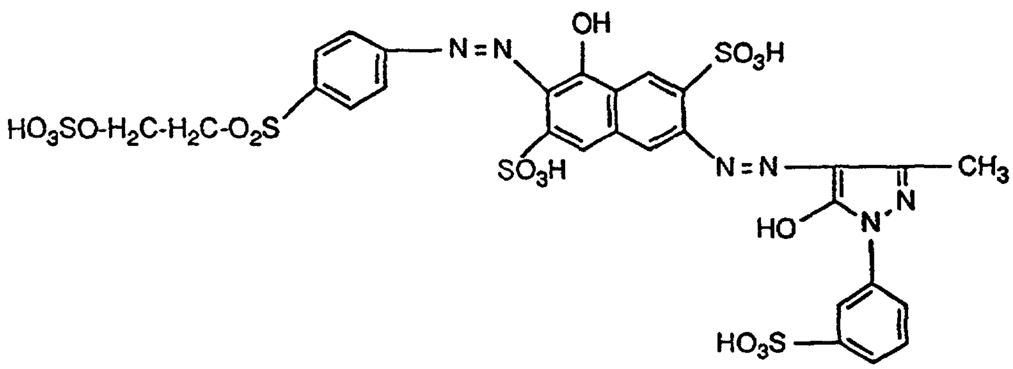

entspricht, als rötliches Pulver, welches Cellulose in orangen Tönen mit guten Allgemeinechtheiten färbt.

Beispiel 15: 18 Teile 1,3,5-Trichlortriazin werden in 100 Teilen Eis/Wasser-Gemisch angeschlämmt und mit 3 Teilen Dinatriumhydrogenphosphat gepuffert. Man tropft eine Lösung von 12 Teilen 2-Aminoethansulfonsäure in 25 Teilen Wasser bei 0 bis 5°C zu und hält den pH-Wert durch Zugabe von Natriumhydroxidlösung bei 7. Nach beendeter Kondensation werden 24 Teile 6-Amino-1-hydroxynaphthalin-3-sulfonsäure unter Konstanthaltung des pH-Werts bei 7 mit Natriumhydroxidlösung eingetragen und anschliessend auf Raumtemperatur erwärmen gelassen. Das nach beendeter Umsetzung erhaltene Biskondensat wird in die Diazolösung 1 gemäss Beispiel 1 getropft und der pH-Wert des Reaktionsgemisches durch Zugabe von Natriumhydroxidlösung allmählich auf 7 erhöht. Man rührt, bis die Kupplung komplett ist, befreit dann die Farbstofflösung dialytisch von Salz und dampft sie im Vakuum ein. Man erhält die Verbindung, die in Form der freien Säure der Formel

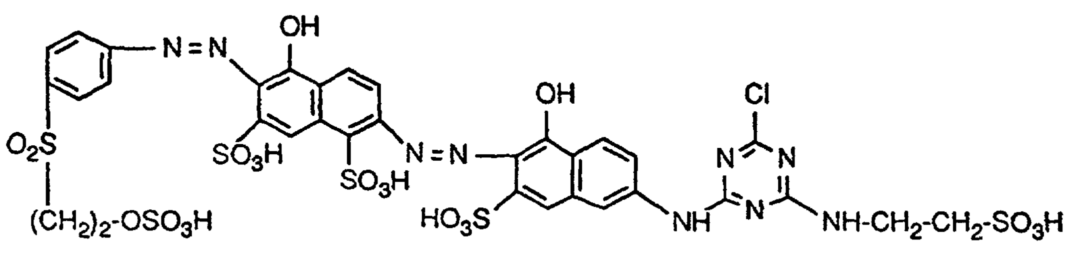

entspricht, als rotes Pulver, welches Cellulose in roten Tönen mit guten Allgemeinechtheiten färbt.

Beispiel 15a: Verfährt man wie im Beispiel 15 angegeben und verwendet anstelle von 24 Teilen 6-Amino-1-hydroxynaphthalin-3-sulfonsäure 26 Teile 6-Methylamino-1-hydroxynaphthalin-3-sulfonsäure, erhält man die Verbindung der Formel

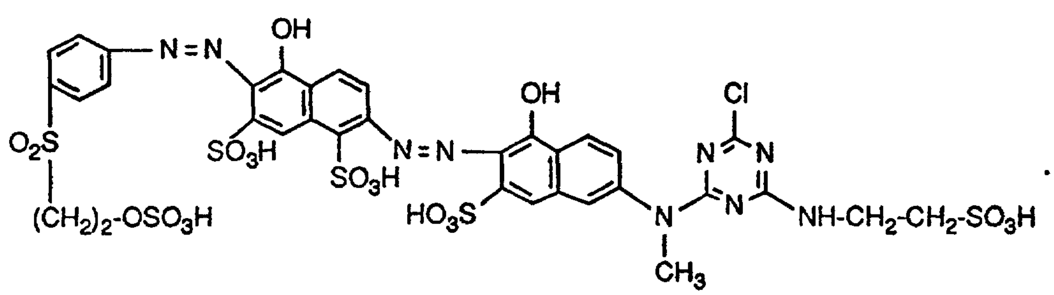

Beispiel 16: 18 Teile 1,3,5-Trichlortriazin werden in 100 Teilen Eis/Wasser-Gemisch angeschlämmt und mit 3 Teilen Dinatriumhydrogenphosphat gepuffert. Man tropft eine Lösung von 24 Teilen 1-Amino-8-hydroxynaphthalin-4,6-disulfonsäure in 100 Teilen Wasser zu und hält die Temperatur bei 0 bis 5°C. Nach beendeter Kondensation wird die Diazolösung 9 gemäss Beispiel 9 zugesetzt und der pH-Wert durch Zugabe von Natriumhydroxidlösung auf 7 erhöht. Man lässt unter Konstanthaltung des pH-Werts auf Raumtemperatur erwärmen und rührt, bis die Kupplung beendet ist. Anschliessend gibt man 7,5 Teile 2-N-Methylaminoethanol zu, erhöht den pH-Wert auf 9 und rührt bei diesem pH-Wert, bis die Kondensation vollständig ist. Die Farbstofflösung wird dialytisch von Salz befreit und im Vakuum eingedampft. Man erhält die Verbindung, die in Form der freien Säure der Formel

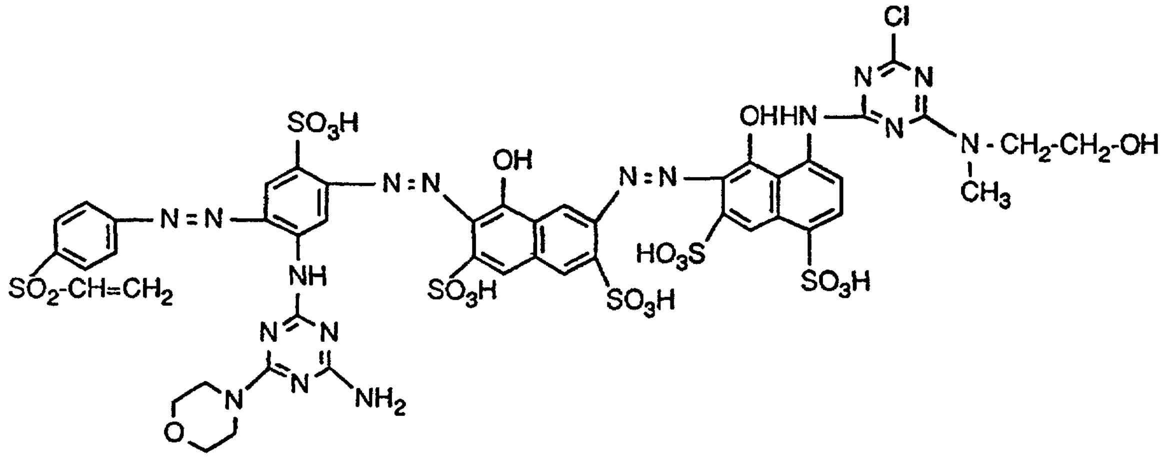

entspricht, als dunkles Pulver, welches Cellulose in marineblauen Tönen mit guten Allgemeinechtheiten färbt.

Beispiele 17-19: Verfährt man wie im Beispiel 16 beschrieben und verwendet anstelle der Diazolösung 9 eine äquivalente Menge der Diazolösungen gemäss Beispiel 10, 11 oder 12, werden die Verbindungen der Formel

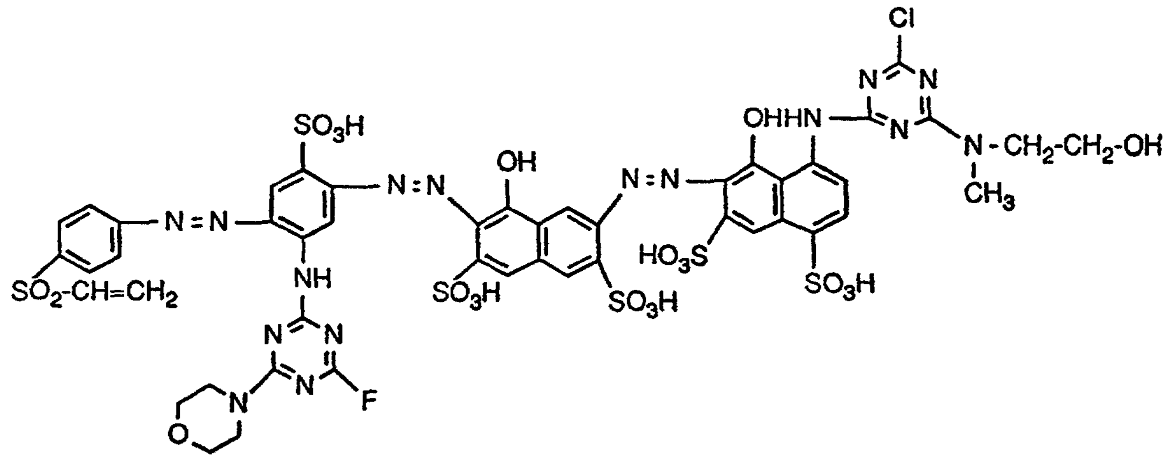

(17)

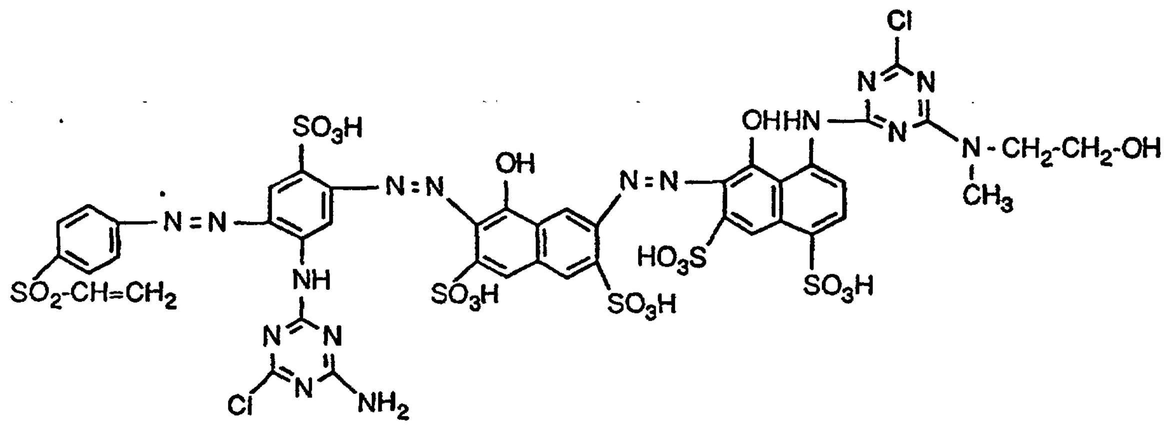

(18) und

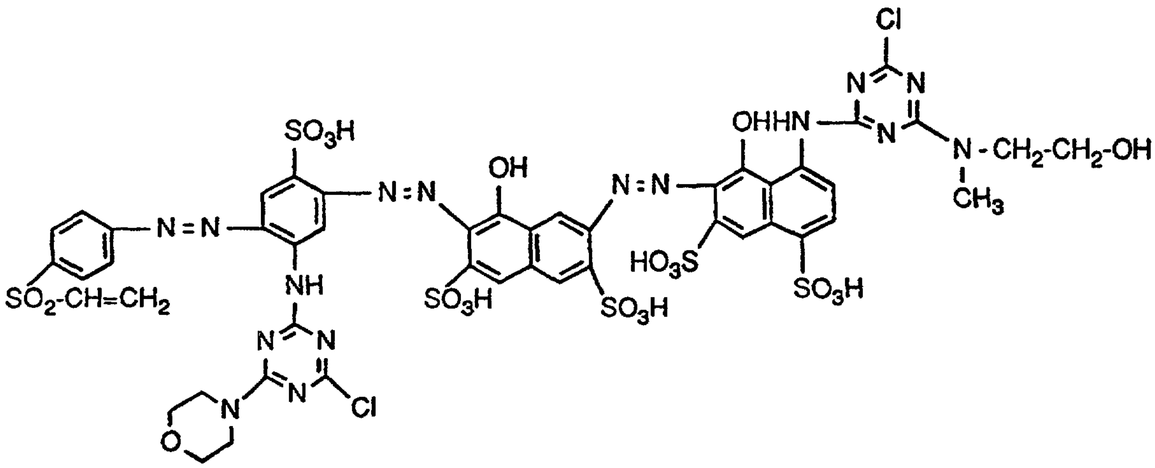

(19)

erhalten, die jeweils Cellulose in marineblauen Tönen mit guten Allgemeinechtheiten färben.

Beispiel 20: 28 Teile 2-(4-Aminophenylsulfonyl)-ethyl-hydrogensulfat werden wie im Beispiel 1 beschrieben diazotiert und die erhaltene Diazolösung bei 0 bis 5°C zu einer Suspension von 32 Teilen 1-Amino-8-hydroxynaphthalin-3,6-disulfonsäure in 100 Teilen Wasser getropft. Man lässt auf Raumtemperatur erwärmen und rührt, bis die Kupplung vollständig ist. Danach wird auf 5 bis 10°C abgekühlt, der pH-Wert durch Zugabe von Natriumhydroxidlösung auf 3 bis 4 erhöht und die Diazolösung 1 gemäss Beispiel 1 unter Konstanthaltung des pH-Werts langsam zugetropft. Nach beendeter Kupplung wird die Farbstofflösung dialytisch von Salz befreit und im Vakuum eingedampft. Man erhält die Verbindung, die in Form der freien Säure der Formel

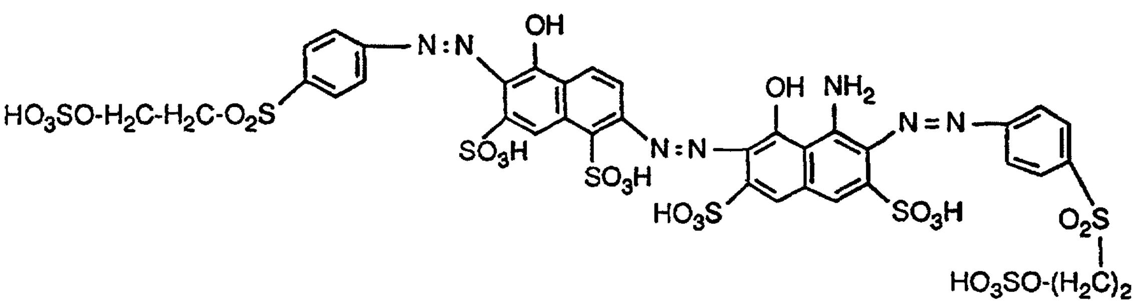

entspricht, als dunkles Pulver, welches Cellulose in marineblauen Tönen mit guten Allgemeinechtheiten färbt.

Beispiel 21: 28 Teile 2-(4-Aminophenylsulfonyl)-ethyl-hydrogensulfat werden wie im Beispiel 1 beschrieben diazotiert und die erhaltene Diazolösung bei 0 bis 5°C zu einer Suspension von 32 Teilen 1-Amino-8-hydroxynaphthalin-3,6-disulfonsäure in 100 Teilen Wasser getropft. Man lässt auf Raumtemperatur erwärmen und rührt, bis die Kupplung vollständig ist. Danach wird auf 5 bis 10°C abgekühlt, der pH-Wert durch Zugabe von Natriumhydroxidlösung auf 3 bis 4 erhöht und die Diazolösung 3 gemäss Beispiel 3 unter Konstanthaltung des pH-Werts langsam zugetropft. Nach beendeter Kupplung wird die Farbstofflösung dialytisch von Salz befreit und im Vakuum eingedampft. Man erhält die Verbindung, die in Form der freien Säure der Formel

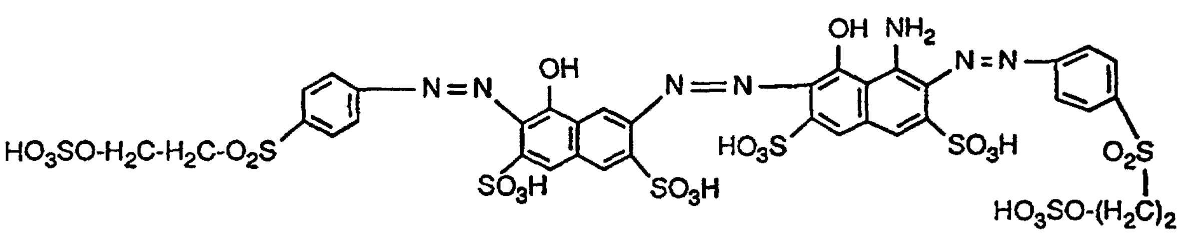

entspricht, als dunkles Pulver, welches Cellulose in marineblauen Tönen mit guten Allgemeinechtheiten färbt.

Beispiel 22: 18 Teile 1,3,5-Trichlortriazin werden in 100 Teilen Eis/Wasser-Gemisch angeschlämmt und mit 3 Teilen Dinatriumhydrogenphosphat gepuffert. Man tropft eine mit Natriumhydroxidlösung neutralisierte Lösung von 27 Teilen 4,6-Diaminobenzol-1,3-disulfonsäure in 100 Teilen Wasser zu, stellt einen pH-Wert von 4,5 ein und hält diesen durch Zugabe von Natriumhydroxidlösung konstant. Nach beendeter Kondensation gibt man 16 Teile konz. Salzsäure dazu, kühlt auf 0 bis 5°C ab und tropft langsam 7 Teile Natriumnitrit, gelöst in 25 Teilen Wasser, zu. Am Ende der Diazotierung wird das überschüssiges Nitrit mit Sulfaminsäure zerstört. Zu der erhaltenen Diazolösung wird bei 0 bis 5°C eine Suspension von 32 Teilen 1-Amino-8-hydroxynaphthalin-3,6-disulfonsäure in 100 Teilen Wasser zugetropft, anschliessend 14 Teile 2-N-Methylaminoethansulfonsäure zugegeben und der pH-Wert langsam auf 6,5 erhöht. Sind die Reaktionen beendet, tropft man die Diazolösung 6 gemäss Beispiel 6 hinzu und hält den pH-Wert konstant bei 6,5. Nach beendeter Kupplung wird die Farbstofflösung dialytisch von Salz befreit und im Vakuum eingedampft. Man erhält die Verbindung, die in Form der freien Säure der Formel

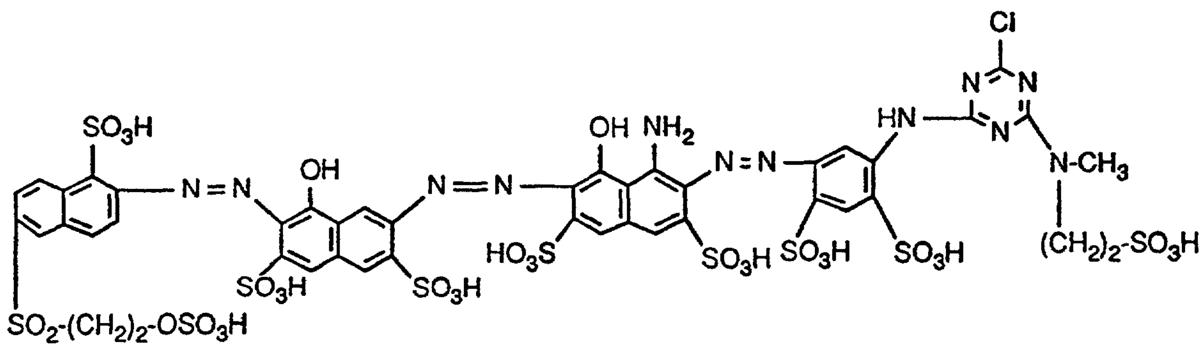

entspricht, als dunkles Pulver, welches Cellulose in oliven Tönen mit guten Allgemeinechtheiten färbt.

Färbevorschrift I 2 Teile des gemäss Beispiel 10 erhaltenen Farbstoffes werden in 400 Teilen Wasser gelöst; dazu gibt man 1500 Teile einer Lösung, die pro Liter 53 g Natriumchlorid enthält. In dieses Färbebad geht man bei 40°C mit 100 Teilen Baumwollgewebe ein. Nach 45 Minuten werden 100 Teile einer Lösung, die pro Liter 16 g Natriumhydroxyd und 20 g kalziniertes Soda enthält, zugegeben. Die Temperatur des Färbebades wird weitere 45 Minuten bei 40°C gehalten. Danach wird die gefärbte Ware gespült, während einer Viertelstunde mit einem nichtionogenen Waschmittel kochend geseift, nochmals gespült und getrocknet.

Färbevorschrift II 2 Teile des gemäss Beispiel 11 erhaltenen Reaktivfarbstoffes werden in 400 Teilen Wasser gelöst; dazu gibt man 1500 Teile einer Lösung die pro Liter 53 g Natriumchlorid enthält. In dieses Färbebad geht man bei 35°C mit 100 Teilen eines Baumwollgewebes ein. Nach 20 Minuten werden 100 Teile einer Lösung, die pro Liter 16 g Natriumhydroxyd und 20 g kalziniertes Soda enthält, zugegeben. Die Temperatur des Färbebades wird weitere 15 Minuten bei 35°C gehalten. Danach wird die Temperatur innerhalb von 20 Minuten auf 60°C erhöht. Die Temperatur wird weitere 35 Minuten bei 60°C gehalten. Danach wird gespült, während einer Viertelstunde mit einem nichtionogenen Waschmittel kochend geseift, nochmals gespült und getrocknet.

Färbevorschrift III 8 Teile des gemäss Beispiel 12 erhaltenen Reaktivfarbstoffes werden in 400 Teilen Wasser gelöst; dazu gibt man 1400 Teile einer Lösung, die pro Liter 100 g Natriumsulfat enthält. In dieses Färbebad geht man bei 25°C mit 100 Teilen eines Baumwollgewebes ein. Nach 10 Minuten werden 200 Teile einer Lösung, die pro Liter 150 g Trinatriumphosphat enthält, zugegeben. Danach wird die Temperatur des Färbebades innerhalb von 10 Minuten auf 60°C erhöht. Die Temperatur wird weitere 90 Minuten auf 60°C gehalten. Danach wird gespült, während einer Viertelstunde mit einem nichtionogenen Waschmittel kochend geseift, nochmals gespült und getrocknet.

Färbevorschrift IV 4 Teile des gemäss Beispiel 13 erhaltenen Reaktivfarbstoffes werden in 50 Teilen Wasser gelöst. Dazu gibt man 50 Teile einer Lösung, die pro Liter 5 g Natriumhydroxyd und 20 g kalziniertes Soda enthält. Mit der erhaltenen Lösung wird ein Baumwollgewebe foulardiert, so dass es um 70 % seines Gewichts zunimmt, und dann auf eine Kaule aufgewickelt. Das Baumwollgewebe wird so während 3 Stunden bei Raumtemperatur gelagert. Danach wird die gefärbte Ware gespült, während einer Viertelstunde mit einem nichtionogenen Waschmittel kochend geseift, nochmals gespült und getrocknet.

Färbevorschrift V 6 Teile des gemäss Beispiel 14 erhaltenen Reaktivfarbstoffes werden in 50 Teilen Wasser gelöst. Dazu gibt man 50 Teile einer Lösung, die pro Liter 16 g Natriumhydroxyd und 0,04 Liter Wasserglas (38°bé) enthält. Mit der erhaltenen Lösung wird ein Baumwollgewebe foulardiert, so dass es um 70 % seines Gewichts zunimmt, und dann auf eine Kaule aufgewickelt. Das Baumwollgewebe wird so während 10 Stunden bei Raumtemperatur gelagert Danach wird die gefärbte Ware gespült, während einer Viertelstunde mit einem nichtionogenen Waschmittel

kochend geseift, nochmals gespült und getrocknet.

Färbevorschrift VI 2 Teile des gemäss Beispiel 13 erhaltenen Reaktivfarbstoffes werden unter Zusatz von 0,5 Teilen m-nitrobenzolsulfonsaurem Natrium in 100 Teilen Wasser gelöst. Mit der erhaltenen Lösung wird ein Baumwollgewebe imprägniert, so dass es um 75 % seines Gewichts zunimmt, und dann getrocknet. Dann imprägniert man das Gewebe mit einer 20°C warmen Lösung, die pro Liter 4 g Natriumhydroxyd und 300 g Natriumchlorid enthält, quetscht auf 75 % Gewichtszunahme ab, dämpft die Färbung während 30 Sekunden bei 100 bis 102°C, spült, seift während einer Viertelstunde in einer 0,3%igen kochenden Lösung eines nichtionogenen Waschmittels, spült und trocknet.

Druckvorschrift I 3 Teile des gemäss Beispiel 15 erhaltenen Reaktivfarbstoffes werden unter schnellem Rühren in 100 Teile einer Stammverdickung, enthaltend 50 Teile 5%ige Natriumalginatverdickung, 27,8 Teile Wasser, 20 Teile Harnstoff, 1 Teil m-nitrobenzolsulfonsaures Natrium sowie 1,2 Teile Natriumhydrogencarbonat, eingestreut. Mit der so erhaltenen Druckpaste bedruckt man ein Baumwollgewebe, trocknet und dämpft den erhaltenen bedruckten Stoff 2 Minuten bei 102°C in gesättigtem Dampf. Das bedruckte Gewebe wird dann gespült, gegebenenfalls kochend geseift und nochmals gespült, und anschliessend getrocknet.

Druckvorschrift II 5 Teile des gemäss Beispiel 16 erhaltenen Reaktivfarbstoffes werden unter schnellem Rühren in 100 Teile einer Stammverdickung, enthaltend 50 Teile 5%ige Natriumalginatverdickung, 36,5 Teile Wasser, 10 Teile Harnstoff, 1 Teil m-nitrobenzolsulfonsaures Natrium sowie 2,5 Teile Natriumhydrogencarbonat, eingestreut. Mit der so erhaltenen Druckpaste, deren Stabilität den technischen Anforderungen entspricht, bedruckt man ein Baumwollgewebe, trocknet und dämpft den erhaltenen bedruckten Stoff 8 Minuten bei 102°C in gesättigtem Dampf. Das bedruckte Gewebe wird dann gespült, gegebenenfalls kochend geseift und nochmals gespült, und anschliessend getrocknet.

**Patentansprüche**

1. Verbindungen der Formel

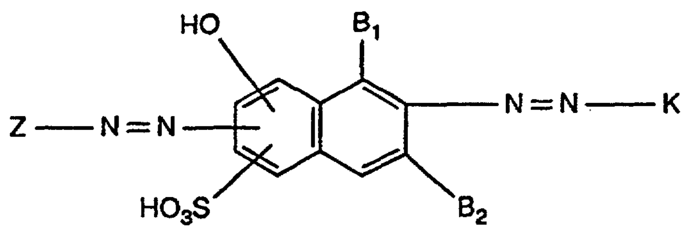

(1),

worin einer der Reste $B_1$ und $B_2$ Wasserstoff und der andere Sulfo ist, K für den Rest einer Kupplungskomponente der Naphthylreihe oder der heterocyclischen Reihe steht und Z den Rest einer hydroxygruppenfreien Diazokomponente der Benzol- oder Naphthalinreihe oder den Rest einer Monoazoverbindung bedeutet, und worin mindestens einer der Reste K oder Z eine faserreaktive Gruppe ausgewählt aus der Gruppe

$$-SO_2-Y \quad (2a),$$

$$-CONR_1-(CH_2)_n-SO_2-Y \quad (2b)$$

und

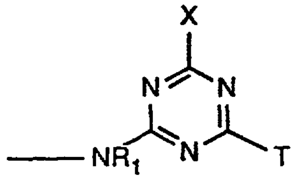

(2c)

aufweist, worin X Halogen, 3-Carboxypyridin-1-yl oder 3-Carbamoylpyridin-1-yl bedeutet, T unabhängig die Bedeutung von X hat oder für Hydroxy, $C_1$-$C_4$-Alkoxy, Phenoxy, $C_1$-$C_4$-Alkylthio, Morpholino, gegebenenfalls durch nicht-reaktive Reste substituiertes Amino oder für einen Reaktivrest der Formel

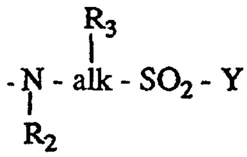

(3a),

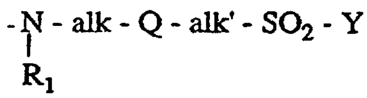

(3b),

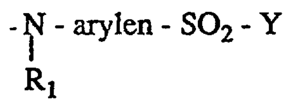

(3c),

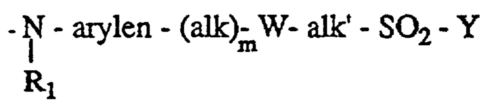

(3d),

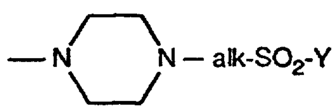

(3e)

oder

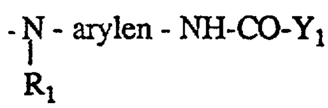

(3f)

steht,

$R_2$ Wasserstoff, gegebenenfalls durch Hydroxy, Sulfo, Sulfato, Carboxy oder Cyano substituiertes $C_1$-$C_4$-Alkyl oder einen Rest

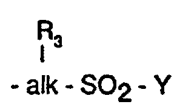

bedeutet,
$R_3$ Wasserstoff, Hydroxy, Sulfo, Sulfato, Carboxy, Cyano, Halogen, $C_1$-$C_4$-Alkoxycarbonyl, $C_1$-$C_4$-Alkanoyloxy, Carbamoyl oder die Gruppe $-SO_2$-Y ist,
alk und alk' unabhängig voneinander $C_1$-$C_6$-Alkylen sind,

arylen einen unsubstituierten oder durch Sulfo, Carboxy, $C_1$-$C_4$-Alkyl, $C_1$-$C_4$-Alkoxy oder Halogen substituierten Phenylen- oder Naphthylenrest bedeutet
$Y_1$ für eine Gruppe $-CHX_2-CH_2X_2$ oder $-CX_2=CH_2$ steht und $X_2$ Chlor oder Brom bedeutet,
Q den Rest -O- oder $-NR_1-$ bedeutet,
W für eine Gruppe $-SO_2-NR_2-$, $-CONR_2-$ oder $-NR_2CO-$ steht,
m die Zahl 0 oder 1 ist
$R_1$ Wasserstoff oder $C_1$-$C_4$-Alkyl ist,
Y Vinyl oder einen Rest $-CH_2-CH_2-U$ bedeutet und U eine alkalisch abspaltbare Abgangsgruppe ist, und
n für eine ganze Zahl von 1 bis 6 steht.

**2.** Verbindungen gemäss Anspruch 1 der Formel

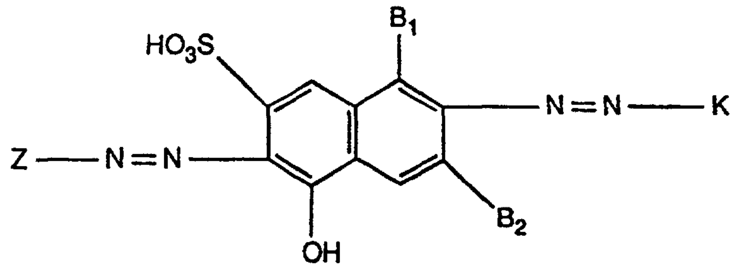

(1a)

oder

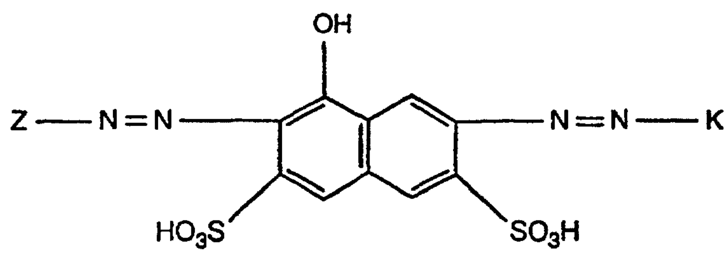

(1b),

worin einer der Reste $B_1$ und $B_2$ Wasserstoff und der andere Sulfo sind und die Variablen K und Z die im Anspruch 1 genannte Bedeutung haben.

**3.** Verbindungen gemäss Anspruch 1 oder 2, **dadurch gekennzeichnet**, dass mindestens einer der Reste K und Z eine faserreaktive Gruppe der im Anspruch 1 angegebenen Formel (2a), (2b) oder (2c) aufweist, worin Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl bedeutet, $R_1$ Wasserstoff ist; n die Zahl 2 oder 3 bedeutet, X Halogen ist und T $C_1$-$C_4$-Alkoxy, Hydroxy, Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, oder einen Reaktivrest der im Anspruch 1 angegebenen Formel (3a) bis (3f) bedeutet, worin W eine Gruppe der Formel -CONH- oder -NHCO-, $R_1$, $R_2$ und $R_3$ je Wasserstoff, Q der Rest -O- oder -NH-, alk und alk' unabhängig voneinander je Ethylen oder Propylen, arylen unsubstituiertes oder durch Methyl, Methoxy, Carboxy oder Sulfo substituiertes Phenylen, $Y_1$ $-CHBr-CH_2Br$ oder $-CBr=CH_2$ und m die Zahl 0 sind und Y die oben angegebene Bedeutung hat.

**4.** Verbindungen gemäss einem der Ansprüche 1 bis 3, **dadurch gekennzeichnet**, dass K der Rest einer Kupplungskomponente der Naphthalin-, Phenylazonaphthalin-, 4-Alkyl-6-hydroxypyridon-(2)-, 2,5-Diamino-4-alkylpyridin-, 1-Arylpyrazolon-(5)- oder 1-Aryl-5-aminopyrazol-Reihe ist.

**5.** Verbindungen gemäss einem der Ansprüche 1 bis 4, **dadurch gekennzeichnet**, dass K einen oder mehrere gleiche oder verschiedene Substituenten aus der Gruppe $C_1$-$C_{12}$-Alkyl, $C_1$-$C_8$-Alkoxy, im Alkylteil durch Hydroxy, $C_1$-

$C_4$-Alkoxy oder Sulfato substituiertes $C_1$-$C_4$-Alkoxy, $C_2$-$C_8$-Acylamino, Amino, gegebenenfalls im Alkylteil durch Hydroxy, Sulfo, Sulfato oder $C_1$-$C_4$-Alkoxy substituiertes N-Mono- oder N,N-Di-$C_1$-$C_4$-Alkylamino, gegebenenfalls im Phenylteil durch Methyl, Methoxy, Halogen oder Sulfo substituiertes Phenylamino, gegebenenfalls im Alkylteil durch Hydroxy, Sulfo oder Sulfato oder im Phenylteil gegebenenfalls durch Methyl, Methoxy, Halogen oder Sulfo substituiertes N-$C_1$-$C_4$-Alkyl-N-phenylamino, gegebenenfalls durch Sulfo substituiertes Naphthylamino, $C_2$-$C_8$-Alkanoyl, Benzoyl, $C_1$-$C_4$-Alkoxycarbonyl, $C_1$-$C_4$-Alkylsulfonyl, Phenyl- oder Naphthylsulfonyl, Trifluormethyl, Nitro, Cyano, Hydroxyl, Halogen, Carbamoyl, N-$C_1$-$C_4$-Alkylcarbamoyl, Sulfamoyl, N-$C_1$-$C_4$-Alkylsulfamoyl, N-(β-Hydroxyethyl)-sulfamoyl, N,N-Di-(β-hydroxyethyl)-sulfamoyl, N-Phenylsulfamoyl, Ureido, Carboxy, Sulfomethyl, Sulfo, Sulfato, Rest der Formel

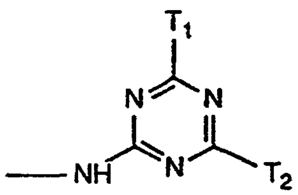

(2c'),

worin $T_1$ und $T_2$ unabhängig voneinander je für Hydroxy, $C_1$-$C_4$-Alkoxy, Morpholino oder gegebenenfalls durch nicht-reaktive Reste substituiertes Amino stehen, und faserreaktiver Rest der im Anspruch 1 angegebenen Formel (2a), (2b) oder der Formel (2c) enthält.

6. Verbindungen gemäss einem der Ansprüche 1 bis 5, **dadurch gekennzeichnet**, dass K für einen Rest der Formel

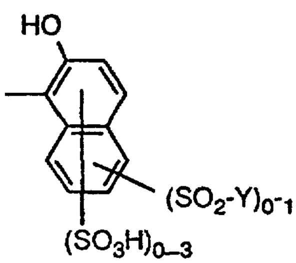

(4a'),

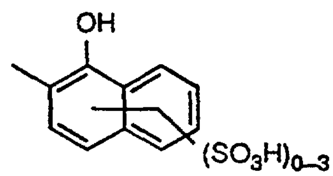

(4a''),

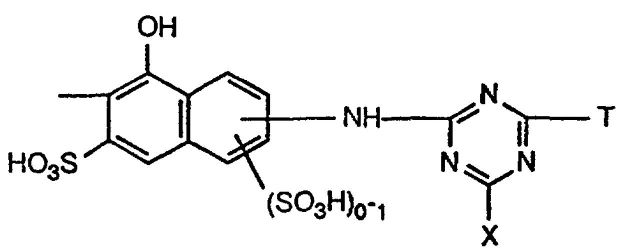

, (4c')

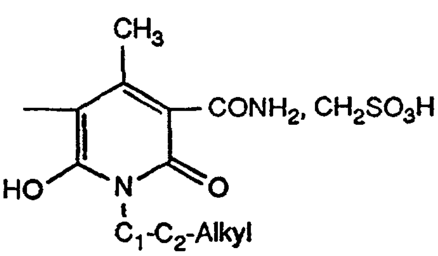

(4d'),

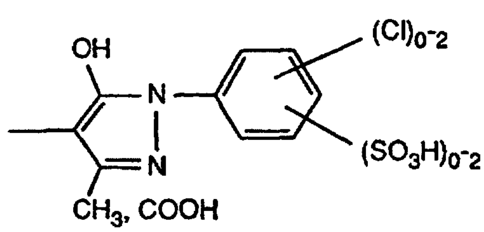

(4f'),

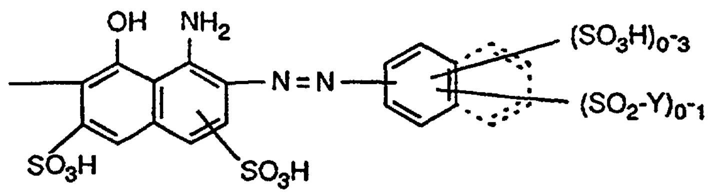 (4h')

oder

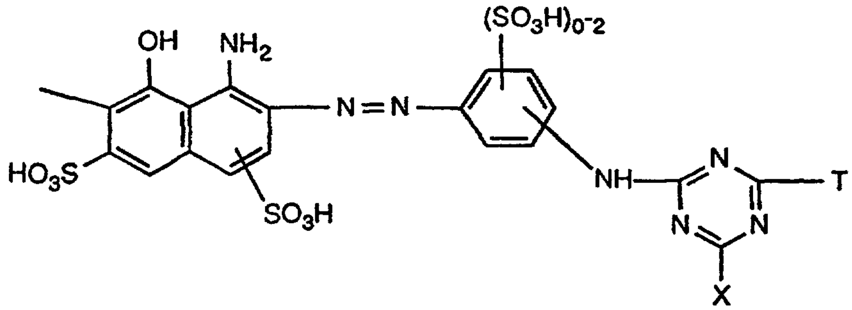 (4h")

steht, worin X Chlor oder Fluor ist, T Amino, N-Mono- oder N,N-Di-$C_1$-$C_4$-Alkylamino, worin das Alkyl unsubstituiert oder durch Hydroxy, Sulfato oder Sulfo substituiert ist, Morpholino oder Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, bedeutet und Y die im Anspruch 1 angegebene Bedeutung hat.

**7.** Verbindungen gemäss Anspruch 6, **dadurch gekennzeichnet**, dass K für einen Rest der Formel (4c'), (4h') oder (4h") steht.

**8.** Verbindungen gemäss einem der Ansprüche 1 bis 7, **dadurch gekennzeichnet**, dass Z einen Phenyl-, Naphthyl-, oder Phenylazophenylrest bedeutet, der eine faserreaktive Gruppe der im Anspruch 1 angegebenen Formel (2a) und/oder (2c) trägt, worin Y Vinyl oder β-Sulfatoethyl, $R_1$ Wasserstoff, X Chlor oder Fluor und T Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, bedeuten, und weiter unsubstituiert oder durch Methyl, Ethyl, Methoxy, Ethoxy, Chlor, Sulfo oder einen Rest der im Anspruch 5 angegebenen Formel (2c') substituiert ist.

**9.** Verbindungen gemäss einem der Ansprüche 1 bis 8, **dadurch gekennzeichnet**, dass Z einen Rest der Formel

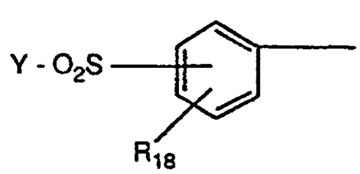 (5a),

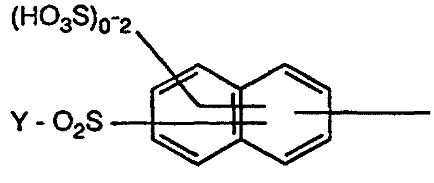 (5b)

oder

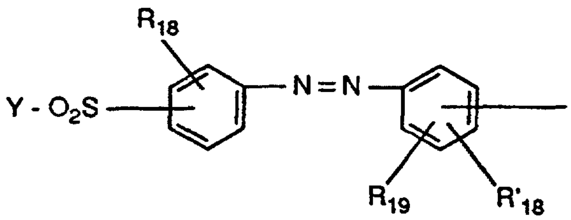

(5c)

bedeutet, worin $R_{18}$ und $R'_{18}$ unabhängig voneinander je Wasserstoff, Sulfo, Methyl, Ethyl, Methoxy, Ethoxy oder Chlor bedeuten, $R_{19}$ Wasserstoff, Sulfo oder ein Rest der im Anspruch 1 angegebenen Formel (2c) oder der im Anspruch 5 angegebenen Formel (2c') ist und Y die im Anspruch 1 genannte Bedeutung hat.

**10.** Verbindungen gemäss Anspruch 1 der Formel

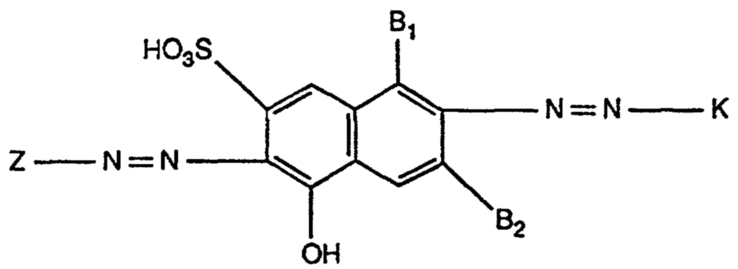

(1a)

oder

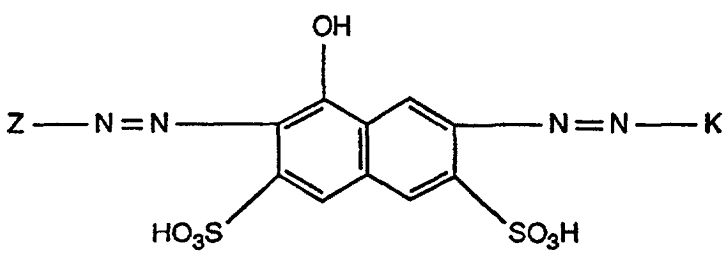

(1b),

worin einer der Reste $B_1$ und $B_2$ Wasserstoff und der andere Sulfo bedeuten,
K für einen Rest der Formel

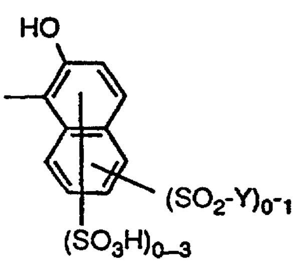

(4a'),

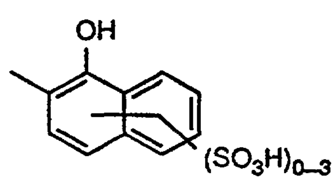

(4a''),

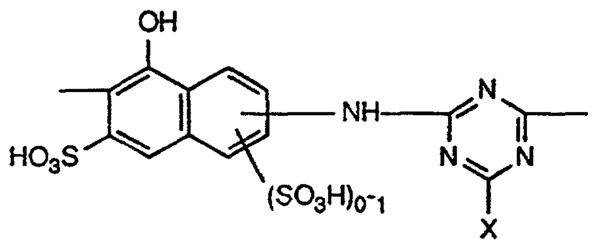

, (4c')

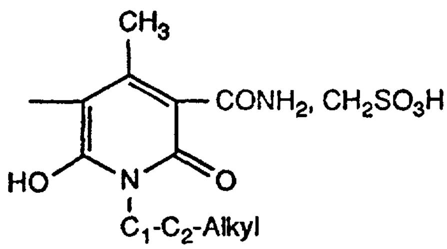

(4d'),

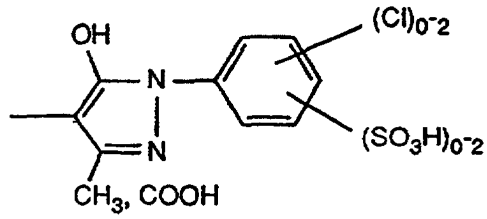

(4f'),

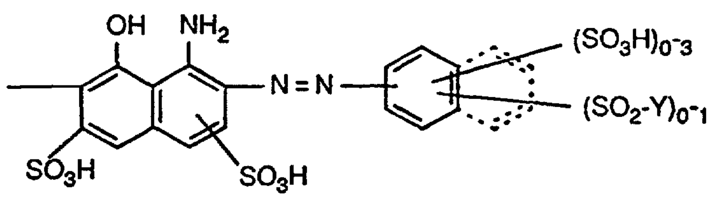

(4h')

oder

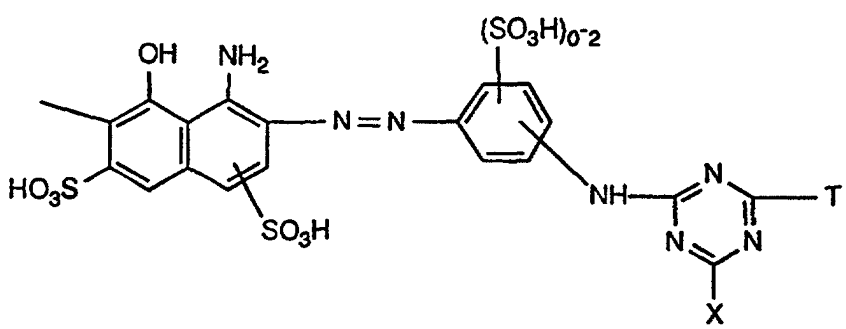

(4h'')

steht, worin X Chlor oder Fluor ist, T Amino, N-Mono- oder N,N-Di-$C_1$-$C_4$-Alkylamino, worin das Alkyl unsubstituiert oder durch Hydroxy, Sulfato oder Sulfo substituiert ist, Morpholino oder Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, bedeutet,

Z einen Rest der Formel

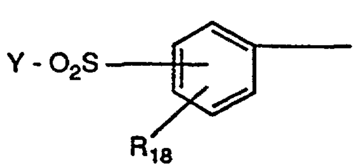

(5a),

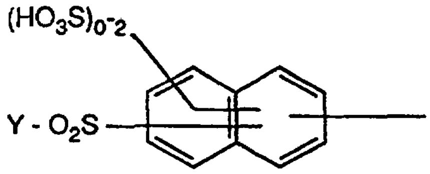

(5b)

oder

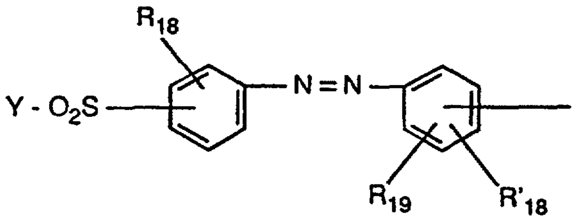

(5c)

bedeutet, worin $R_{18}$ und $R'_{18}$ unabhängig voneinander je Wasserstoff, Sulfo, Methyl, Ethyl, Methoxy, Ethoxy oder Chlor bedeuten, $R_{19}$ Wasserstoff, Sulfo, ein Rest der im Anspruch 1 angegebenen Formel (2c), worin $R_1$ Wasserstoff, X Chlor oder Fluor und T Amino, N-Mono- oder N,N-Di-$C_1$-$C_4$-Alkylamino, worin das Alkyl unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, bedeuten oder ein Rest der im Anspruch 5 angegebenen Formel (2c') ist, worin $T_1$ und $T_2$ unabhängig voneinander je für Amino, N-Mono- oder N,N-Di-$C_1$-$C_4$-Alkylamino, worin das Alkyl unsubstituiert oder durch Hydroxy, Sulfato oder Sulfo substituiert ist, Morpholino oder Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist, stehen, und
Y Vinyl oder β-Sulfatoethyl bedeutet.

**11.** Verfahren zur Herstellung von Verbindungen der Formel (1) gemäss Anspruch 1, **dadurch gekennzeichnet**, dass man in etwa 1 Moläquivalent einer Verbindung der Formel

$$Z\text{-}NH_2 \qquad (6)$$

diazotiert und mit etwa einem Moläquivalent einer Verbindung der Formel

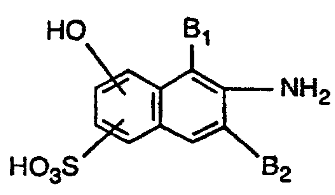

(7)

kuppelt, und die erhaltene Monoazoverbindung erneut diazotiert und mit einer Verbindung der Formel

$$K\text{-}H \qquad (8)$$

kuppelt, wobei Z, $B_1$, $B_2$ und K jeweils die im Anspruch 1 angegebene Bedeutung haben.

**12.** Verwendung von Verbindungen der Formel (1) als faserreaktive Farbstoffe zum Färben oder Bedrucken von hydroxylgruppenhaltigen oder stickstoffhaltigen Fasermaterialien.

**13.** Verwendung gemäss Anspruch 12, **dadurch gekennzeichnet**, dass man cellulosehaltige Fasermaterialien, insbesondere baumwollhaltige Fasermaterialien, färbt oder bedruckt.

## Claims

**1.** A compound of the formula

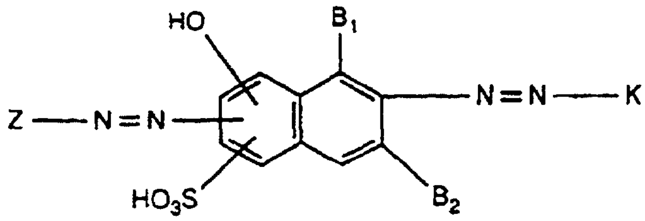

(1),

in which
one of the radicals $B_1$ and $B_2$ is hydrogen and the other is sulfo,

K is the radical of a coupling component of the naphthyl series or of the heterocyclic series and
Z is the radical of a diazo component, which is free from hydroxyl groups, of the benzene or naphthalene series or the radical of a monoazo compound,

and in which at least one of the radicals K or Z contains a fibre-reactive group chosen from the group consisting of

$$-SO_2-Y \qquad (2a),$$

$$-CONR_1-(CH_2)_n-SO_2-Y \qquad (2b)$$

and

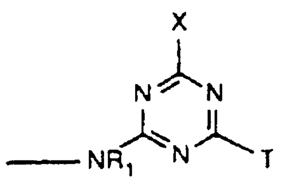

(2c)

in which

X is halogen, 3-carboxypyridin-1-yl or 3-carbamoylpyridin-1-yl,
T independently is as defined for X or is hydroxyl, $C_1$-$C_4$alkoxy, phenoxy, $C_1$-$C_4$alkylthio, morpholino, amino which is unsubstituted or substituted by non-reactive radicals, or a reactive radical of the formula

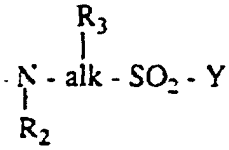

(3a),

or

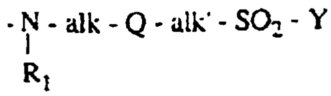 (3b).

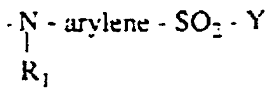 (3c).

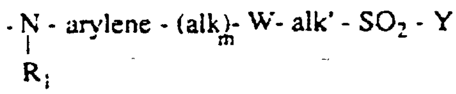 (3d).

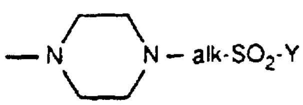 (3e)

or

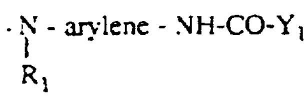 (3f).

$R_2$ is hydrogen, $C_1$-$C_4$alkyl which is unsubstituted or substituted by hydroxyl, sulfo, sulfato, carboxyl or cyano, or a radical

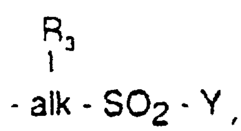

$R_3$ is hydrogen, hydroxyl, sulfo, sulfato, carboxyl, cyano, halogen, $C_1$-$C_4$alkoxycarbonyl, $C_1$-$C_4$alkanoyloxy, carbamoyl or the group $-SO_2-Y$,
alk and alk' independently of one another are $C_1$-$C_6$alkylene,
arylene is a phenylene or naphthylene radical which is unsubstituted or substituted by sulfo, carboxyl, $C_1$-$C_4$alkyl, $C_1$-$C_4$alkoxy or halogen,
$Y_1$ is a group $-CHX_2-CH_2X_2$ or $-CX_2=CH_2$ and
$X_2$ is chlorine or bromine,
Q is the radical -O- or $-NR_1-$,
W is a group $-SO_2-NR_2-$, $-CONR_2-$ or $-NR_2CO-$,
m is the number 0 or 1,
$R_1$ is hydrogen or $C_1$-$C_4$alkyl,
Y is vinyl or a radical $-CH_2-CH_2-U$ and
U is a leaving group which can be split off under alkaline conditions, and
n is an integer from 1 to 6.

**2.** A compound according to claim 1, of the formula

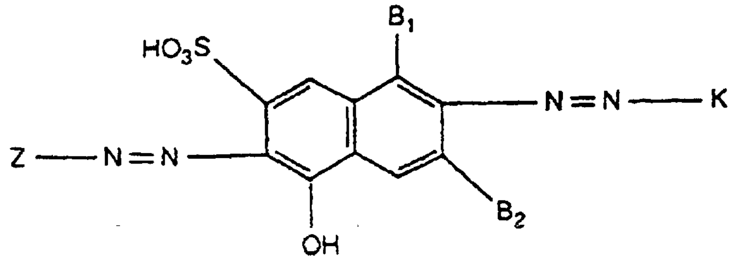

(1a)

or

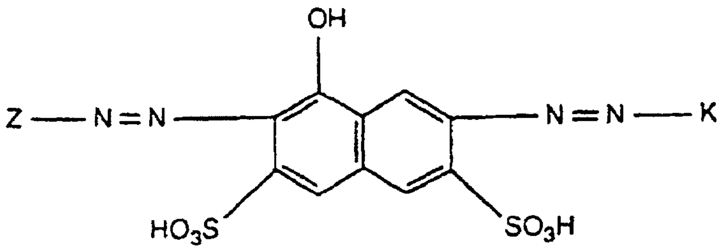

(1b),

in which

one of the radicals $B_1$ and $B_2$ is hydrogen and the other is sulfo and
the variables K and Z are as defined in claim 1.

**3.** A compound according to claim 1 or 2, in which at least one of the radicals K and Z contains a fibre-reactive group of the formula (2a), (2b) or (2c) defined in claim 1, in which

Y is vinyl, β-bromo- or β-chloroethyl, β-acetoxyethyl, β-benzoyloxyethyl, β-phosphatoethyl, β-sulfatoethyl or β-thiosulfatoethyl,
$R_1$ is hydrogen,
n is the number 2 or 3,
X is halogen and
T is $C_1$-$C_4$alkoxy, hydroxyl, amino, N-mono- or N,N-di-$C_1$-$C_4$alkylamino, in which the alkyl is unsubstituted or substituted by hydroxyl, sulfato or sulfo, morpholino, phenylamino or N-$C_1$-$C_4$alkyl-N-phenylamino, in which the phenyl is in each case unsubstituted or substituted by sulfo, carboxyl, acetylamino, methyl or methoxy, or a reactive radical of the formula (3a) to (3f) defined in claim 1, in which
W is a group of the formula -CONH- or -NHCO-,
$R_1$, $R_2$ and $R_3$ are each hydrogen,
Q is the radical -O- or -NH-,
alk and alk' independently of one another are each ethylene or propylene,
arylene is phenylene which is unsubstituted or substituted by methyl, methoxy, carboxyl or sulfo,
$Y_1$ is $-CHBr-CH_2Br$ or $-CBr=CH_2$ and
m is the number 0 and
Y is as defined above.

**4.** A compound according to any one of claims 1 to 3, in which
K is the radical of a coupling component of the naphthalene, phenylazonaphthalene, 4-alkyl-6-hydroxypyrid-2-one, 2,5-diamino-4-alkylpyridine, 1-arylpyrazol-5-one or 1-aryl-5-aminopyrazole series.

**5.** A compound according to any one of claims 1 to 4, in which
K contains one or more identical or different constituents from the group consisting of $C_1$-$C_{12}$alkyl, $C_1$-$C_8$alkoxy,

$C_1$-$C_4$alkoxy which is substituted in the alkyl part by hydroxyl, $C_1$-$C_4$alkoxy or sulfato, $C_2$-$C_8$acylamino, amino, N-mono- or N,N-di-$C_1$-$C_4$alkylamino which is unsubstituted or substituted in the alkyl part by hydroxyl, sulfo, sulfato or $C_1$-$C_4$alkoxy, phenylamino which is unsubstituted or substituted in the phenyl part by methyl, methoxy, halogen or sulfo, N-$C_1$-$C_4$alkyl-N-phenylamino which is unsubstituted or substituted in the alkyl part by hydroxyl, sulfo or sulfato or unsubstituted or substituted in the phenyl part by methyl, methoxy, halogen or sulfo, naphthylamino which is unsubstituted or substituted by sulfo, $C_2$-$C_8$alkanoyl, benzoyl, $C_1$-$C_4$alkoxycarbonyl, $C_1$-$C_4$alkylsulfonyl, phenyl- or naphthylsulfonyl, trifluoromethyl, nitro, cyano, hydroxyl, halogen, carbamoyl, N-$C_1$-$C_4$alkylcarbamoyl, sulfamoyl, N-$C_1$-$C_4$alkylsulfamoyl, N-(β-hydroxyethyl)sulfamoyl, N,N-di-(β-hydroxyethyl)-sulfamoyl, N-phenylsulfamoyl, ureido, carboxyl, sulfomethyl, sulfo, sulfato, a radical of the formula

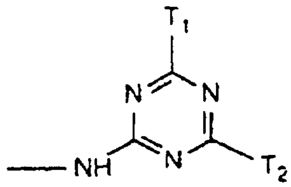

(2c'),

in which
$T_1$ and $T_2$ independently of one another are each hydroxyl, $C_1$-$C_4$alkoxy, morpholino or amino which is unsubstituted or substituted by non-reactive radicals,
and a fibre-reactive radical of the formula (2a), (2b) or of the formula (2c) defined in claim 1.

**6.** A compound according to any one of claims 1 to 5, in which
K is a radical of the formula

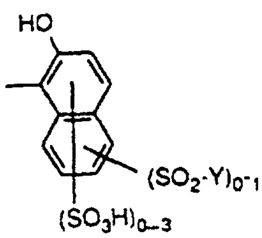

(4a'),

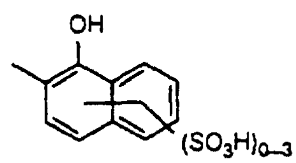

(4a''),

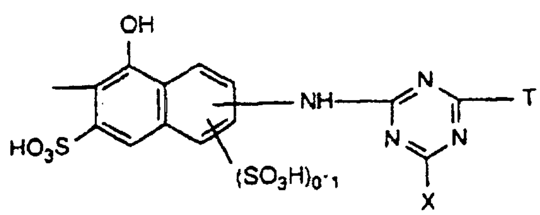

(4c'),

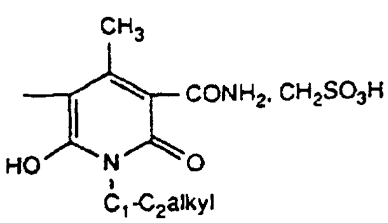

(4d'),

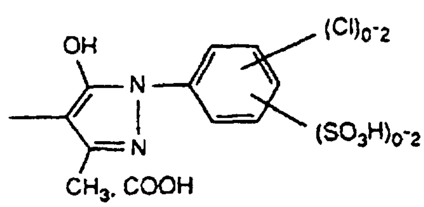

(4f').

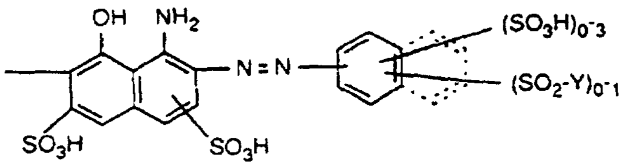

(4h')

or

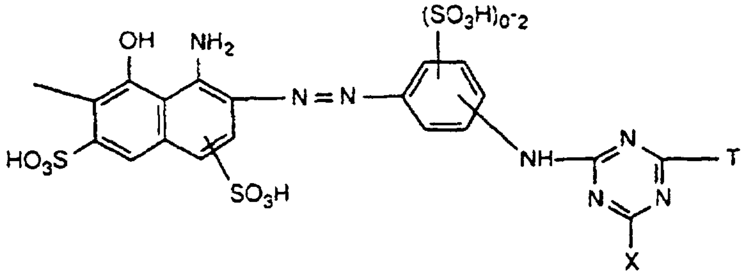

(4h'')

in which

X is chlorine or fluorine,
T is amino, N-mono- or N,N-di-$C_1$-$C_4$alkylamino, in which the alkyl is unsubstituted or substituted by hydroxyl, sulfato or sulfo, morpholino or phenylamino or N-$C_1$-$C_4$alkyl-N-phenylamino, in which the phenyl is in each case unsubstituted or substituted by sulfo, carboxyl, acetylamino, methyl or methoxy, and
Y is as defined in claim 1.

**7.** A compound according to claim 6, in which
K is a radical of the formula (4c'), (4h') or (4h'').

**8.** A compound according to any one of claims 1 to 7, in which

Z is a phenyl, naphthyl or phenylazophenyl radical which carries a fibre-reactive group of the formula (2a) and/or (2c) defined in claim 1, in which
Y is vinyl or β-sulfatoethyl,
$R_1$ is hydrogen,
X is chlorine or fluorine and
T is amino, N-mono- or N,N-di-$C_1$-$C_4$alkylamino, in which the alkyl is unsubstituted or substituted by hydroxyl, sulfato or sulfo, morpholino, phenylamino or N-$C_1$-$C_4$alkyl-N-phenylamino, in which the phenyl is in each case unsubstituted or substituted by sulfo, carboxyl, acetylamino, methyl or methoxy,

and is furthermore unsubstituted or substituted by methyl, ethyl, methoxy, ethoxy, chlorine, sulfo or a radical of the formula (2c') defined in claim 5.

**9.** A compound according to any one of claims 1 to 8, in which

Z is a radical of the formula

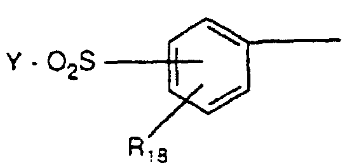

(5a),

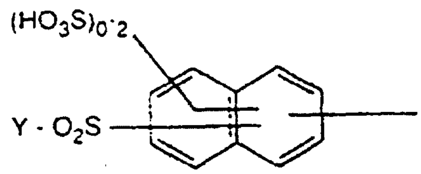

(5b)

or

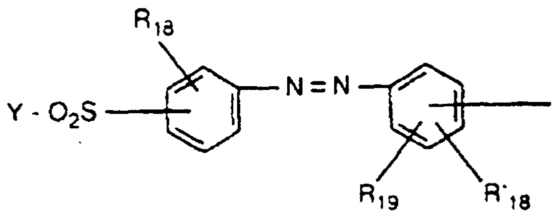

(5c),

in which
$R_{18}$ and $R'_{18}$ independently of one another are each hydrogen, sulfo, methyl, ethyl, methoxy, ethoxy or chlorine,
$R_{19}$ is hydrogen, sulfo or a radical of the formula (2c) defined in claim 1 or of the formula (2c') defined in claim 5 and
Y is as defined in claim 1.

**10.** A compound according to claim 1, of the formula

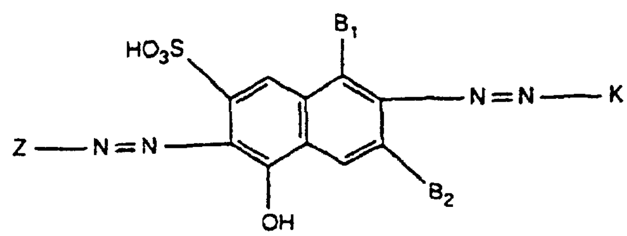

(1a)

or

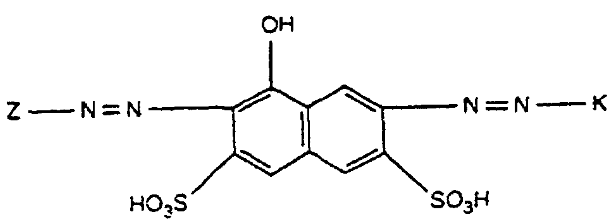

(1b)

in which
one of the radicals $B_1$ and $B_2$ is hydrogen and the other is sulfo,

K is a radical of the formula

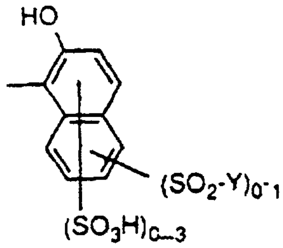 (4a'),

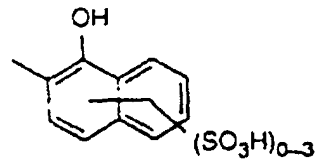 (4a''),

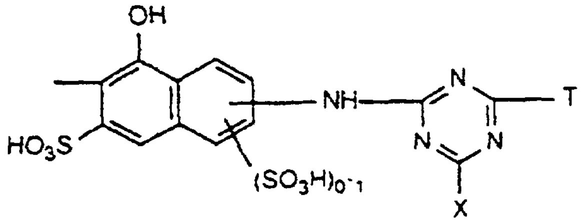 (4c'),

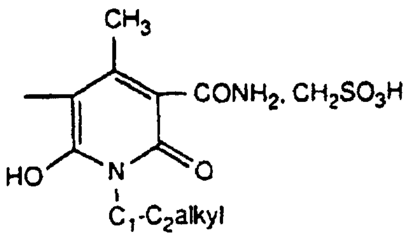 (4d'),

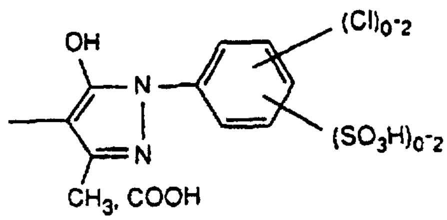 (4f'),

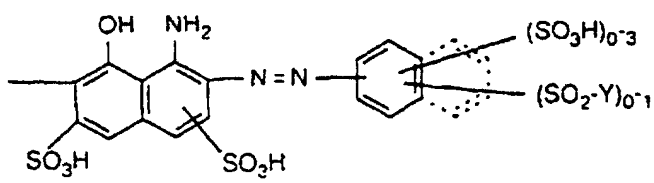 (4h')

or

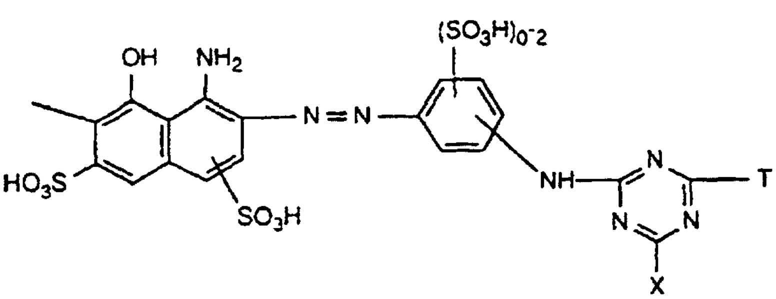 (4h'')

in which
X is chlorine or fluorine,
T is amino, N-mono- or N,N-di-$C_1$-$C_4$alkylamino, in which the alkyl is unsubstituted or substituted by hydroxyl, sulfato or sulfo, morpholino or phenylamino or N-$C_1$-$C_4$alkyl-N-phenylamino, in which the phenyl is in each case unsubstituted or substituted by sulfo, carboxyl, acetylamino, methyl or methoxy, and

Z is a radical of the formula

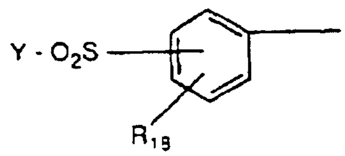

(5a),

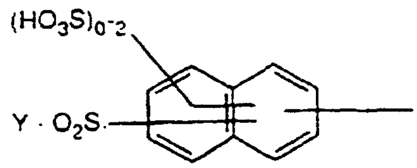

(5b)

or

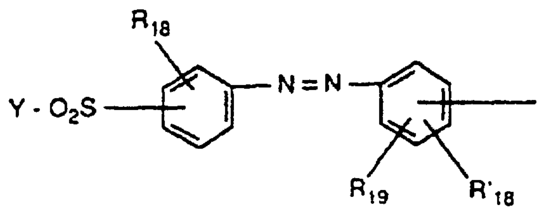

(5c).

in which
$R_{18}$ and $R'_{18}$ independently of one another are each hydrogen, sulfo, methyl, ethyl, methoxy, ethoxy or chlorine,
$R_{19}$ is hydrogen, sulfo or a radical of the formula (2c) defined in claim 1, in which
$R_1$ is hydrogen,
X is chlorine or fluorine and
T is amino, N-mono- or N,N-di-$C_1$-$C_4$alkylamino, in which the alkyl is unsubstituted or substituted by hydroxyl, sulfato or sulfo, morpholino, phenylamino or N-$C_1$-$C_4$alkyl-N-phenylamino, in which the phenyl is in each case unsubstituted or substituted by sulfo, carboxyl, acetylamino, methyl or methoxy,
or a radical of the formula (2c') defined in claim 5, in which
$T_1$ and $T_2$ independently of one another are each amino, N-mono- or N,N-di-$C_1$-$C_4$alkylamino, in which the alkyl is unsubstituted or substituted by hydroxyl, sulfato or sulfo, morpholino or phenylamino or N-$C_1$-$C_4$alkyl-N-phenylamino, in which the phenyl is in each case unsubstituted or substituted by sulfo, carboxyl, acetylamino, methyl or methoxy, and
Y is vinyl or β-sulfatoethyl.

**11.** A process for the preparation of a compound of the formula (1) according to claim 1, which comprises diazotizing about 1 molar equivalent of a compound of the formula

$$Z\text{-}NH_2 \qquad (6),$$

coupling the diazotization product with about one molar equivalent of a compound of the formula

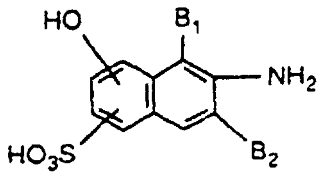

(7)

and diazotizing the resulting monoazo compound again and coupling the diazotization product to a compound of the formula

$$K\text{-}H \qquad (8)$$

in which
Z, $B_1$, $B_2$ and K are each as defined in claim 1.

**12.** The use of a compound of the formula (1) as a fibre-reactive dye for dyeing or printing fibre materials containing hydroxyl groups or containing nitrogen.

**13.** The use according to claim 12, wherein cellulosic fibre materials, in particular fibre materials containing cotton, are dyed or printed.

## Revendications

**1.** Composés de formule

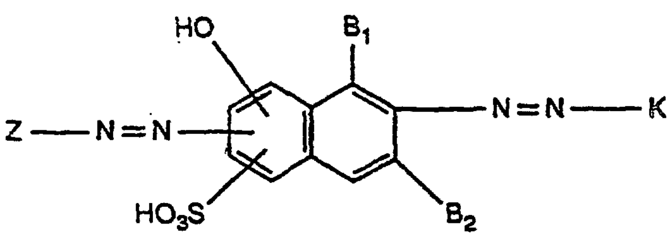

(1),

où l'un des restes $B_1$ et $B_2$ représente un atome d'hydrogène et l'autre un groupe sulfo, K représente le reste d'un composant de copulation de la série naphtyle ou de la série hétérocyclique et Z représente le reste d'un composant de diazotation ne contenant pas de groupes hydroxyle de la série du benzène ou du naphtalène, ou le reste d'un composé monoazoïque et où au moins l'un des restes K ou Z présente un groupe réactif sur la fibre pris dans le groupe

$$-SO_2-Y \qquad (2a),$$

$$-CONR_1-(CH_2)_n-SO_2-Y \qquad (2b)$$

et

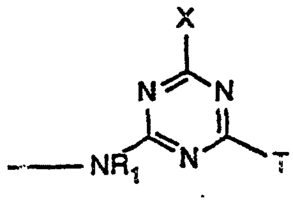

(2c),

où

X représente un atome d'halogène, des groupes 3-carboxypyridin-1-yle ou 3-carbamoylpyridin-1-yle, T possède indépendamment la signification de X ou représente des groupes hydroxy, alkoxy en $C_1$-$C_4$, phénoxy, (alkyl en $C_1$-$C_4$)-thio, morpholino, amino éventuellement substitué par des restes réactifs ou représente un reste réactif de formule

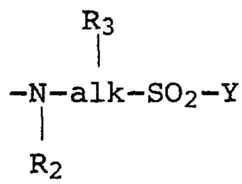

(3a),

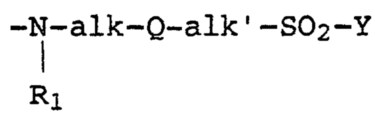

(3b),

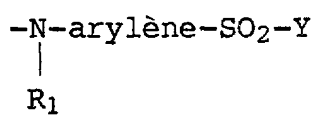

(3c),

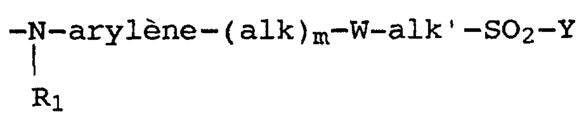

(3d),

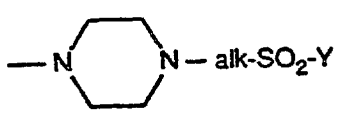

(3e)

ou

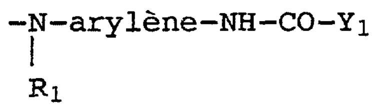 (3f).

$R_2$ représente un atome d'hydrogène, un groupe alkyle en $C_1$-$C_4$ éventuellement substitué par des substituants hydroxy, sulfo, sulfato, carboxy ou cyano ou un reste

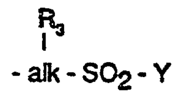 ,

$R_3$ représente un atome d'hydrogène, des groupes hydroxy, sulfo, sulfato, carboxy, cyano, halogène, (alkoxy en $C_1$-$C_4$)-carbonyle, (alcanoyl en $C_1$-$C_4$)oxy, carbamoyle ou le groupe $-SO_2$-Y,
alk et alk' représentent, indépendamment l'un de l'autre, un groupe alkylène en $C_1$-$C_6$,
arylène signifie un reste phénylène ou naphtylène non substitué ou substitué par des substituants sulfo, carboxy, alkyle en $C_1$-$C_4$, alkoxy en $C_1$-$C_4$ ou halogène,
$Y_1$ représente un groupe $-CHX_2-CH_2X_2$ ou $-CX_2=CH_2$ et $X_2$ représente un atome de chlore ou de brome,
Q représente le reste -O- ou $-NR_1-$,
W représente un groupe $-SO_2-NR_2-$, $-CONR_2-$ ou $-NR_2CO-$,
m vaut 0 ou 1,
$R_1$ représente un atome d'hydrogène ou un groupe alkyle en $C_1$-$C_4$,
Y représente un groupe vinyle ou un reste $-CH_2-CH_2-U$ et
U représente un groupe partant dissociable en milieu alcalin, et
n est un entier de 1 à 6.

**2.** Composés selon la revendication 1 de formule

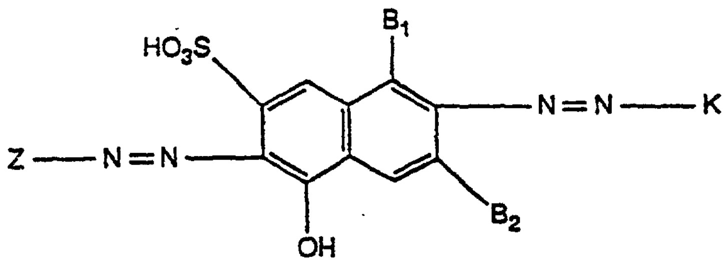 (1a)

ou

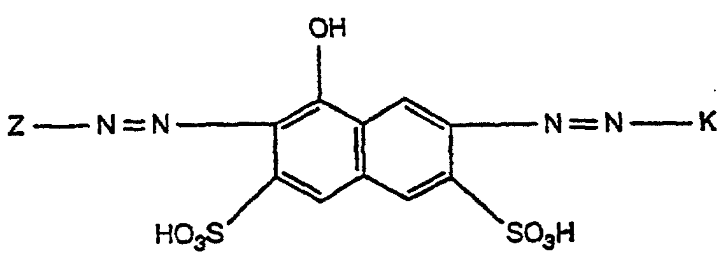 (1b)

où l'un des restes $B_1$ et $B_2$ représente un atome d'hydrogène et l'autre un groupe sulfo et les variables K et Z possèdent la signification donnée à la revendication 1.

**3.** Composés selon la revendication 1 ou 2, **caractérisés en ce que** au moins un des restes K et Z présente un groupe réactif sur la fibre de formule (2a), (2b) ou (2c) donnée à la revendication 1, où Y représente des groupes

vinyle, β-brométhyle ou β-chloréthyle, β-acétoxyéthyle, β-benzoyloxyéthyle, β-phosphatoéthyle, β-sulfatoéthyle ou β-thiosulfatoéthyle, $R_1$ représente un atome d'hydrogène, n vaut 2 ou 3, X représente un atome d'halogène et T des groupes alkoxy en $C_1$-$C_4$, hydroxy, amino, N-mono- ou N,N-di-(alkyl en $C_1$-$C_4$)amino, où le fragment alkyle est non substitué ou substitué par des substituants hydroxy, sulfato ou sulfo, représente des groupes morpholino, phénylamino ou N-(alkyl en $C_1$-$C_4$)-N-phénylamino, où le fragment phényle est chaque fois non substitué ou substitué par des substituants sulfo, carboxy, acétylamino, méthyle ou méthoxy, ou représente un reste réactif de formule (3a) à (3f) donnée à la revendication 1, où W représente un groupe de formule -CONH- ou -NHCO-, $R_1$, $R_2$ et $R_3$ représentent chacun un atome d'hydrogène, Q représente le reste -O- ou -NH-, alk et alk' représentent chacun, indépendamment l'un de l'autre, des groupes éthylène ou propylène, arylène représente un groupe phénylène non substitué ou substitué par des substituants méthyle, méthoxy, carboxy ou sulfo, $Y_1$ représente des groupes $-CHBr-CH_2Br$ ou $-CBr=CH_2$ et m vaut 0 et Y possède la signification donnée ci-dessus.

4. Composés selon l'une des revendications 1 à 3, **caractérisés en ce que** K représente le reste d'un composant de copulation de la série du naphtalène, du phénylazonaphtalène, de la 4-alkyl-6-hydroxypyridone-(2), de la 2,5-diamino-4-alkylpyridine, de la 1-aryl-pyrazolone-(5) ou du 1-aryl-5-aminopyrazole.

5. Composés selon l'une des revendications 1 à 4, **caractérisés en ce que** K contient un ou plusieurs substituants identiques ou différents pris dans le groupe comportant alkyle en $C_1$-$C_{12}$, alkoxy en $C_1$-$C_8$, alkoxy en $C_1$-$C_4$ substitué dans le fragment alkyle par des substituants hydroxy, alkoxy en $C_1$-$C_4$ ou sulfato, représente des groupes (acyl en $C_2$-$C_8$)amino, amino, N-mono- ou N,N-di-(alkyl en $C_1$-$C_4$)amino éventuellement substitué dans le fragment alkyle par des substituants hydroxy, sulfo, sulfato ou alkoxy en $C_1$-$C_4$, un groupe phénylamino éventuellement substitué dans le fragment phényle par des substituants méthyle, méthoxy, halogène ou sulfo, un groupe N-(alkyl en $C_1$-$C_4$)-N-phénylamino éventuellement substitué dans le fragment alkyle par des substituants hydroxy, sulfo ou sulfato, ou dans le fragment phényle par des substituants méthyle, méthoxy, halogène ou sulfo, un groupe naphtylamino éventuellement substitué par un substituant sulfo, des groupes alcanoyle en $C_2$-$C_8$, benzoyle, (alkoxy en $C_1$-$C_4$)-carbonyle, (alkyl en $C_1$-$C_4$)sulfonyle, phényl- ou naphtylsulfonyle, trifluorométhyle, nitro, cyano, hydroxyle, halogène, carbamoyle, N-(alkyl en $C_1$-$C_4$)-carbamoyle, sulfamoyle, N-(alkyl en $C_1$-$C_4$)sulfamoyle, N-(β-hydroxyéthyl)-sulfamoyle, N,N-di-(β-hydroxyéthyl)-sulfamoyle, N-phénylsulfamoyle, uréido, carboxy, sulfométhyle, sulfo, sulfato, un reste de formule

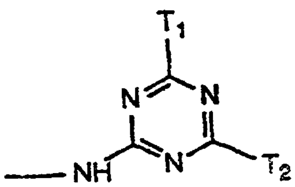

(2c'),

où $T_1$ et $T_2$ représentent chacun, indépendamment l'un de l'autre, des groupes hydroxy, alkoxy en $C_1$-$C_4$, morpholino ou amino éventuellement substitué par des restes non réactifs, et un reste réactif sur la fibre de formule (2a), (2b) ou de formule (2c) donnée à la revendication 1.

6. Composés selon l'une des revendications 1 à 5, **caractérisés en ce que** K représente un reste de formule

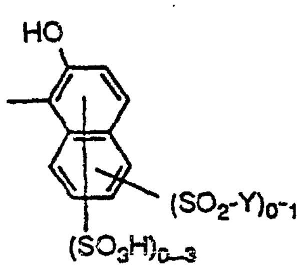

(4a'),

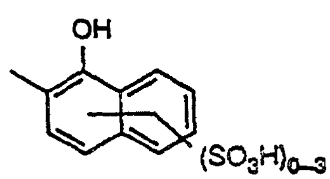

(4a''),

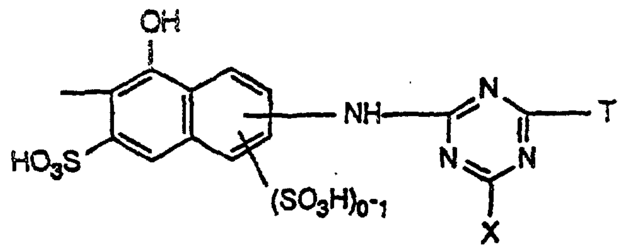

, (4c')

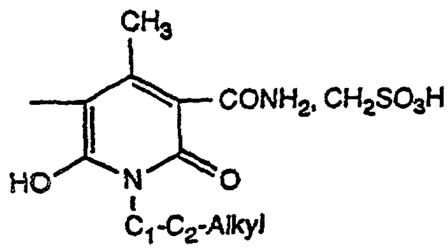

(4d'),

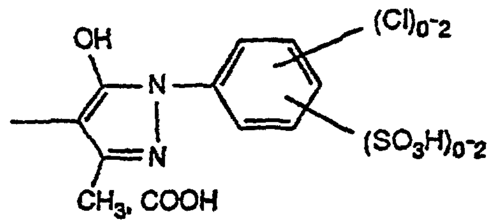

(4f'),

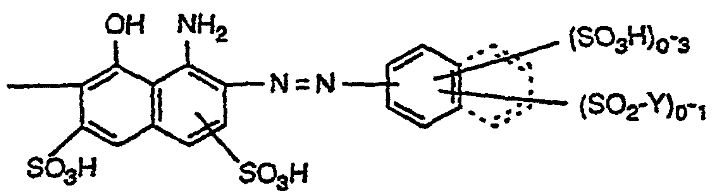

(4h')

ou

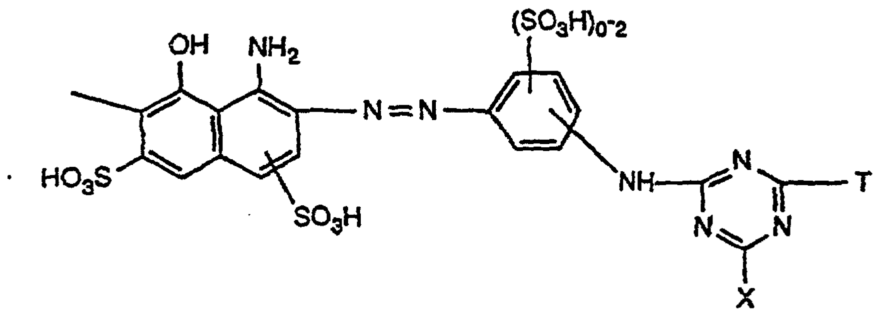

(4h'')

où X représente un atome de chlore ou de fluor, T représente des groupes amino, N-mono- ou N,N-di(alkyl en $C_1$-$C_4$)amino, où le fragment alkyle est non substitué ou substitué par des substituants hydroxy, sulfato ou sulfo, représente des groupes morpholino ou phénylamino ou N-(alkyl en $C_1$-$C_4$)-N-phénylamino, où le fragment phényle est chaque fois non substitué ou substitué par des substituants sulfo, carboxy, acétylamino, méthyle ou méthoxy, et Y possède la signification donnée à la revendication 1.

**7.** Composés selon la revendication 6 **caractérisés en ce que** K représente un reste de formule (4c'), (4h') ou (4h'').

**8.** Composés selon l'une des revendications 1 à 7, **caractérisés en ce que** Z représente un reste phényle, naphtyle ou phénylazophényle, qui porte un groupe réactif sur la fibre de formule (2a) et/ou (2b) donnée à la revendication 1, où Y représente un groupe vinyle ou β-sulfatoéthyle, $R_1$ représente un atome d'hydrogène, X représente un atome de chlore ou de fluor et T représente des groupes amino, N-mono- ou N,N-di-(alkyl en $C_1$-$C_4$)amino, où le fragment alkyle est non substitué ou substitué par des substituants hydroxy, sulfato ou sulfo, des groupes morpholino, phénylamino ou N-(alkyl en $C_1$-$C_4$)-N-phénylamino, où le fragment phényle est chaque fois non substitué ou substitué par des substituants sulfo, carboxy, acétylamino, méthyle ou méthoxy, et n'est pas substitué davantage ou est substitué par des substituants méthyle, éthyle, méthoxy, éthoxy, chloro, sulfo ou un reste de formule (2c') donnée à la revendication 5.

**9.** Composés selon l'une des revendications 1 à 8, **caractérisés en ce que** Z représente un reste de formule

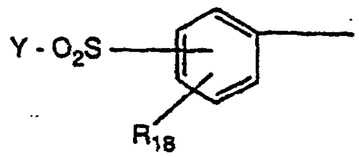

(5a),

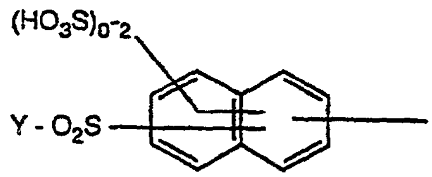

(5b)

ou

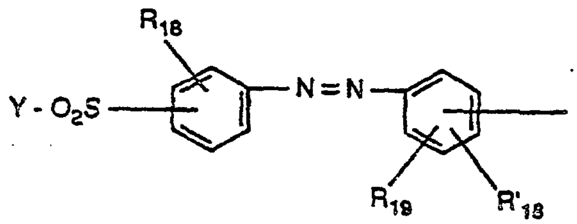

(5c)

où

$R_{18}$ et $R'_{18}$ représentent chacun, indépendamment l'un de l'autre, des atomes d'hydrogène, des groupes sulfo, méthyle, éthyle, méthoxy, éthoxy ou chloro,

$R_{19}$ représente un atome d'hydrogène, un groupe sulfo ou un reste de formule (2c) ou (2c') donnée à la revendication 1 ou de formule (2c') donnée à la revendication 5 et Y possède la signification donnée à la revendication 1.

**10.** Composés selon la revendication 1 de formule

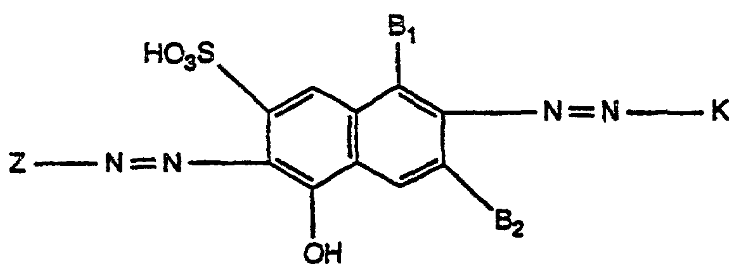

(1a)

ou

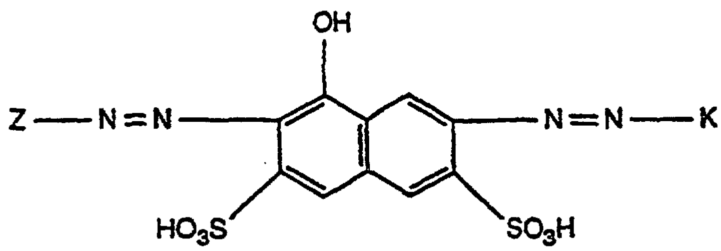

(1b)

où l'un des restes $B_1$ et $B_2$ représente un atome d'hydrogène et l'autre un groupe sulfo,

K représente un reste de formule

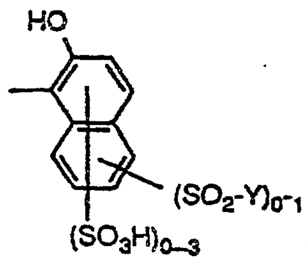

(4a'),

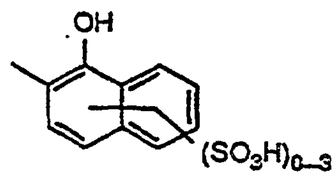

(4a''),

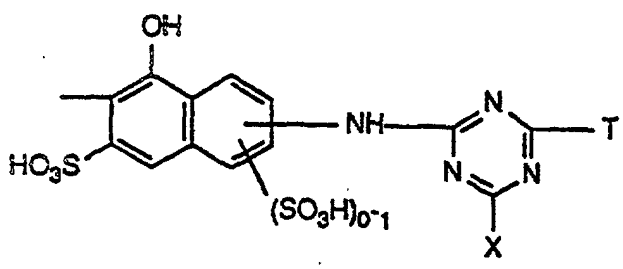

, (4c')

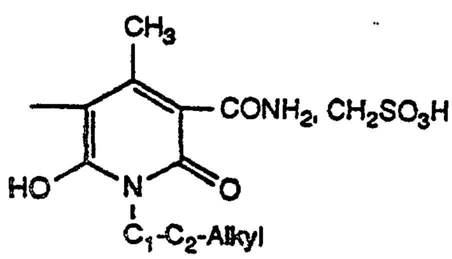

(4d'),

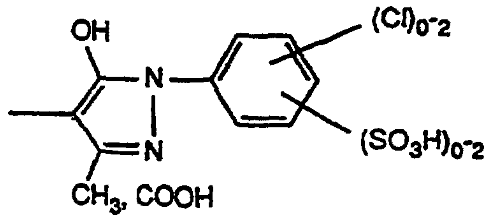

(4f'),

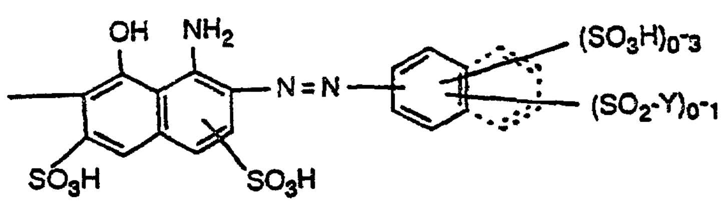

(4h')

ou

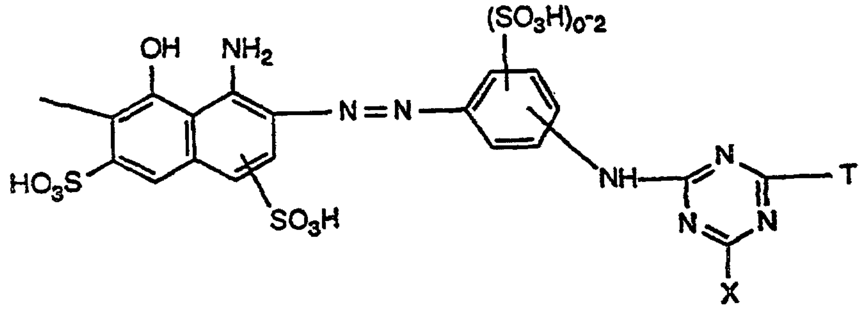

(4h'')

où X représente un atome de chlore ou de fluor, T représente des groupes amino, N-mono- ou N,N-di-(alkyl en $C_1$-$C_4$)amino, où le fragment alkyle. est non substitué ou substitué par des substituants hydroxy, sulfato ou sulfo, des groupes morpholino ou phénylamino ou N-(alkyl en $C_1$-$C_4$)-N-phénylamino, où le fragment phényle est chaque fois non substitué ou substitué par des substituants sulfo, carboxy, acétylamino, méthyle ou méthoxy,

Z représente un reste de formule

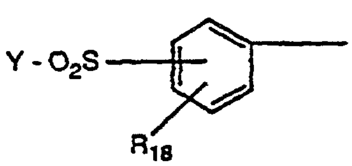

(5a),

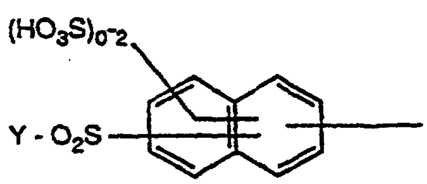

(5b)

ou

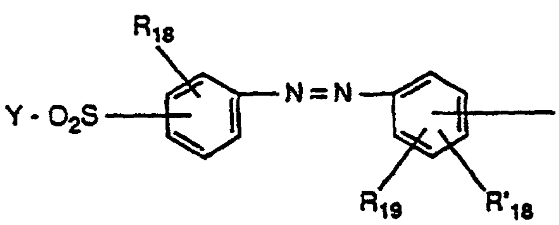

(5c)

où $R_{18}$ et $R'_{18}$ représentent chacun, indépendamment l'un de l'autre, des atomes d'hydrogène, des groupes sulfo, méthyle, éthyle, méthoxy, éthoxy ou chloro, $R_{19}$ représente un atome d'hydrogène, un groupe sulfo ou un reste de formule (2c) donnée à la revendication 1, où $R_1$ représente un atome d'hydrogène, X représente un atome de chlore ou de fluor et T représente des groupes amino, N-mono- ou N,N-di-(alkyl en $C_1$-$C_4$)amino, où le fragment alkyle est non substitué ou substitué par des substituants hydroxy, sulfato ou sulfo, morpholino, phénylamino ou N-(alkyl en $C_1$-$C_4$)-N-phénylamino, où le fragment phényle est chaque fois non substitué ou substitué par des substituants sulfo, carboxy, acétylamino, méthyle ou méthoxy, et Y représente un groupe vinyle ou β-sulfatoéthyle.

**11.** Procédé pour la préparation de composés de formule (1) selon la revendication 1, **caractérisé en ce qu'**on diazote environ 1 équivalent molaire d'un composé de formule

$$Z\text{-}NH_2 \qquad (6)$$

de façon usuelle, par exemple avec du nitrite de sodium en milieu acide, par exemple, acide par l'acide chlorhydrique, et on copule avec environ 1 équivalent molaire d'un composé de formule

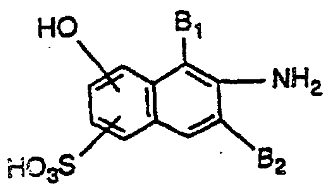

(7),

et on diazote à nouveau le composé manoazoïque obtenu de façon usuelle et on copule avec un composé de formule

$$K\text{-}H \qquad (8),$$

Z, $B_1$, $B_2$ et K ayant chacun la signification donnée à la revendication 1.

**12.** Utilisation de composés de formule (1) en tant que colorants réactifs sur la fibre pour la teinture ou l'impression de matières fibreuses azotées ou contenant des groupes hydroxyle.

**13.** Utilisation selon la revendication 12, **caractérisée en ce qu'**on teint ou on imprime des matières fibreuses cellulosiques, en particulier des matières fibreuses contenant du coton.